# EUROPEAN PATENT SPECIFICATION

(11) **EP 3 297 642 B1**
(45) Date of publication and mention of the grant of the patent: **11.09.2019**
(21) Application number: 16730086.2
(22) Date of filing: 20.05.2016
(51) Int. Cl.: A61K 35/17, C12N 5/0783, C12N 15/113

(54) **SCREENING METHODS FOR TARGETS FOR CANCER THERAPY**
SCREENINGVERFAHREN FÜR TARGETS ZUR KREBSTHERAPIE
PROCÉDÉS DE CRIBLAGE POUR DES CIBLES POUR LA THÉRAPIE DU CANCER

(30) Priority: 22.05.2015 US 201562165784 P
(43) Date of publication of application: 28.03.2018
(73) Proprietor: STCube & Co., Inc., Seoul 06168 (KR); Board of Regents, The University of Texas System, Austin, TX 78701 (US)
(72) Inventor: LIN, Steven, Hsesheng, Pearland, TX 77584 (US); YOO, Stephen, Sunghan, Centreville, VA 20120 (US)
(74) Representative: Jones Day
(86) International application number: PCT/US2016/033651
(87) International publication number: WO 2016/191315

(56) References cited:
- WO-A2-2014/201021
- N. KHANDELWAL ET AL: "A high-throughput RNAi screen for detection of immune-checkpoint molecules that mediate tumor resistance to cytotoxic T lymphocytes", EMBO MOLECULAR MEDICINE, vol. 7, no. 4, 1 April 2015 (2015-04-01), pages 450-463, XP55297296, Weinheim ISSN: 1757-4676, DOI: 10.15252/emmm.201404414
- ANUSHA KALBASI ET AL: "Radiation and immunotherapy: a synergistic combination", JOURNAL OF CLINICAL INVESTIGATION, vol. 123, no. 7, 1 July 2013 (2013-07-01), pages 2756-2763, XP55297249, US ISSN: 0021-9738, DOI: 10.1172/JCI69219
- KIM SUNGJUNE ET AL: "Radiation-induced autophagy potentiates immunotherapy of cancer via up-regulation of mannose 6-phosphate receptor on tumor cells in mice", CANCER IMMUNOLOGY AND IMMUNOTHERAPY, SPRINGER-VERLAG, BERLIN, DE, vol. 63, no. 10, 19 June 2014 (2014-06-19) , pages 1009-1021, XP035394529, ISSN: 0340-7004, DOI: 10.1007/S00262-014-1573-4 [retrieved on 2014-06-19]

## Description

### 1. FIELD

Provided herein are methods for identifying potential targets for cancer therapy.

### 2. BACKGROUND

Tumor microenvironment is intrinsically inhibitory due to the presence of myeloid-derived suppressor cells ("MDSC") and regulatory T cells ("T-reg") that infiltrate the tumor and function to suppress immune responses. In addition, the expression of certain inhibitory molecules on T cells and antigen presenting cells (APCs) can limit effective immune responses. Radiation mediates anti-tumor effects through the induction of tumor cell apoptosis, senescence, autophagy. Radiation can modulate the tumor microenvironment through factors that can serve as targets for cancer therapy. Thus, methods to screen for these factors and to treat cancer by targeting these factors represent unmet needs. The methods provided of the present disclosure meet these needs and provide other related advantages.

WO 2014/201021 discloses a method of identifying a gene from a non-irradiated tumor that inhibits the function of an immunoresponsive T-cell.

Khandelwal et al., EMBO Molecular Medicine, vol. 7: 4, p. 450-463 discloses a high-throughput siRNA-based screening approach that allows identification of ligands in human cancer cells that inhibit CTL-mediated tumor cell killing.

### 3. SUMMARY

In one aspect, the invention provides an *in vitro* method of identifying a target gene for cancer therapy comprising measuring an activity of an unmodified T cell and of a modified T cell; said unmodified T cell and said modified T cell each contacted with the tumor microenvironment from an irradiated tumor-bearing subject; said modified T cell having a candidate gene knocked-down or knocked-out; wherein said candidate gene is identified as a target gene for cancer therapy if said activity is increased comparing said modified T cell to said unmodified T cell.

In one embodiment, said activity is selected from the group consisting of proliferation, cell cycle activation, cell death inhibition, cytokine production, and a cytotoxic activity. In another embodiment, the method comprises measuring said activity of said unmodified T cell and of said modified T cell each contacted with the tumor microenvironment from a non-irradiated tumor-bearing subject; wherein said activity in said unmodified T cell and said modified T cell are substantially the same.

In a second aspect, the invention provides an *in vivo* method of identifying a target gene for cancer therapy comprising: (i) injecting unmodified T cell and modified T cell to an irradiated tumor-bearing non-human test subjects; said modified T cell having a candidate gene knocked-down or knocked-out; and (ii) measuring an activity of said unmodified T cell and of said modified T cell in said non-human test subjects, wherein said activity is proliferation activity or anti-tumor activity; wherein said candidate gene is identified as a target gene for cancer therapy if said activity is increased comparing said modified T cell to said unmodified T cell; optionally wherein said modified T cell is a reference T cell.

In one embodiment, the method further comprises injecting said unmodified T cell and said modified T cell to non-irradiated test subjects, wherein said activity is substantially the same comparing said modified T cell to said unmodified T cell in said non-irradiated test subjects.

In one embodiment, said subject has received tumor-targeted radiation at a dose from 1 Gy to 20 Gy. In another embodiment, said activity is: (i) proliferation activity measured by accumulation of injected T cells; (ii) anti-tumor activity measured by death of tumor cells or the reduction of tumor size; or (iii) anti-tumor activity measured by increased survival of tested subjects. In another embodiment, said test subject is a mouse, preferably wherein said mouse is a two-tumor mouse, wherein only one tumor has received targeted radiation.

Preferably, said activity is anti-tumor activity measured by size reduction of the non-irradiated tumor.

In a third aspect, the invention provides an *in vitro* pool screen method of identifying a target gene for cancer therapy comprising: (i) preparing a starting population of T cells with different genetic modifications, wherein each T cell has at most one candidate gene knocked-down or knocked-out; and (ii) contacting said starting population of T cells with the tumor microenvironment from an irradiated tumor-bearing subject for a period of time to produce a selected population of T cells; wherein the candidate gene that is knocked-down or knocked-out in the selected population of T cells is identified to be the target gene for cancer therapy.

In a fourth aspect, the invention provides an *in vivo* pool screen method of identifying a target gene for cancer therapy comprising: (i) preparing a starting population of T cells with different genetic modifications, wherein each T cell has at most one candidate gene knocked-down or knocked-out; and (ii) injecting said starting population of T cells to an irradiated tumor-bearing non-human test subject, in which said starting population of T cells produces a selected population of T cells; wherein the candidate gene that is knocked-down or knocked-out in the selected population of T cells is identified to be the target gene for cancer therapy.

In one embodiment, said candidate gene is knocked-down or knocked-out in said modified T cell, or said starting population of T cells by: (i) using a transcription activator-like effector nuclease (TALEN); (ii) using CAS9 (CRISPR); or (iii) infecting T cells with a shRNA library, optionally wherein the shRNA library is a lentivirus vector library. Preferably wherein, each T cell of the starting population in part (iii) has at most a shRNA for a candidate gene, and a candidate gene is identified to be a target gene for cancer therapy if the shRNA for the candidate gene is enriched in the selected population of T cells as compared to the starting population of T cells, optionally wherein: (i) a candidate gene is identified to be a target gene for cancer therapy if the shRNA for the candidate gene is enriched in the selected population of T cells obtained from a tumor sample as compared to a non-tumor tissue sample; (ii) a candidate gene is identified as a target gene if the shRNA for said candidate gene is enriched more than 2 fold in the tumor sample compared to the non-tumor tissue sample; or (iii) a candidate gene is identified as a target gene if the shRNA for said candidate gene is enriched more than 3 fold, more than 4 fold, more than 5 fold, more than 10 fold, more than 15 fold, or more than 20 fold in the tumor sample compared to the non-tumor tissue sample, wherein the method optionally comprises quantifying shRNA in the selected population of T cells by NGS.

In another embodiment, said T cells or said starting population of T cells are: (i) naive T cells; (ii) CD8+ T cells; or (iii) isolated from a mouse, optionally wherein said mouse is wild type, or genetically engineered to express a T-cell receptor specific for an antigen not naturally present in mice, optionally wherein said antigen is ovalbumin. In another embodiment, said T cells or said starting population of T cells are labeled, optionally wherein said label is carboxyfluorescein succinimidyl ester (CFSE).

In one embodiment, the method of the third and fourth aspects further comprises isolating said selected population of T cells from a sample of said test subject, optionally wherein: (i) said sample is a tumor sample, optionally wherein said tumor sample has received targeted radiation or the test subject is a two-tumor model, and said tumor sample has not received targeted radiation; or (ii) said sample is a non-tumor sample, optionally wherein said non-tumor tissue sample is a spleen sample or a lymph node sample. In another embodiment, the candidate gene that is knocked-down or knocked-out in the selected population of T cells is identified by NGS of genomic DNAs isolated from the selected population of T cells.

In a fifth aspect, the invention provides an *ex vivo* method for activating a T cell by inhibiting the target gene identified in any one of the preceding aspects or embodiments of said T cell.

Provided herein are *in vitro,* high throughput screening methods for identifying a target for cancer therapy using a T cell suppression assay in combination with radiation treatment.

Also provided herein are *in vitro* screening methods for identifying a target for cancer therapy using a T cell suppression assay in combination with radiation treatment, wherein assays are conducted using target pools which are expressed in cell cultures.

Also provided herein are *in vivo* screening methods for identifying a target for cancer therapy using a T cell suppression assay in combination with radiation treatment in tumor implanted non-human subjects.

In certain embodiments, the *in vitro* and *in vivo* screening methods provided herein can be used in combination, e.g., sequentially conducting an *in vitro* method provided herein, followed by an *in vivo* method provided herein using candidate targets identified from the *in vitro* method.

### 3.1. DEFINITIONS

As used herein, and unless otherwise specified, the articles "a," "an," and "the" refer to one or to more than one of the grammatical object of the article. By way of example, a T cell refers to one T cell or more than one T cells.

As used herein, and unless otherwise specified, the term "cancer" or "cancerous" refers to the physiological condition that is typically characterized by unregulated cell growth in a subject. Examples of cancer include, but are not limited to, hematological cancers and solid tumors.

As used herein, and unless otherwise specified, the term "treat," "treating," "treatment," when used in reference to a cancer patient, refer to an action that reduces the severity of the cancer, or retards or slows the progression of the cancer, including (a) inhibiting the cancer growth, or arresting development of the cancer, and (b) causing regression of the cancer, or delaying or minimizing one or more symptoms associated with the presence of the cancer. Treatment of cancer can involve one or a combination of two or more different types of therapies, including surgery, chemotherapy, radiation therapy, targeted therapy, immune therapy, etc.

Radiation therapy is a type of cancer treatment that uses beams of intense energy to harm or kill cancer cells. Radiation therapy can use X-rays, protons or other types of energy. Radiation therapy in many instances refers to external beam radiation therapy. During this type of radiation, the high-energy beams are generated by a machine outside of the patient's body that aims the beams at a precise point on the tumor. In other types of radiation therapies, such as brachytherapy, the source of radiation can also be placed inside the patient's body.

As used herein, and unless otherwise specified, the term "subject" or "patient" refers to an animal that is the object of treatment, observation and/or experiment. "Animal" includes vertebrates and invertebrates, such as fish, shellfish, reptiles, birds, and, in particular, mammals. "Mammal" includes, but not limited to, mice, rats, rabbits, guinea pigs, dogs, cats, sheep, goats, cows, horses, primates, such as monkeys, chimpanzees, apes, and humans. A subject can refer to an animal for use in *in vivo* tests or experiments. The subject of testing or experimentation can also be referred to a "test subject."

In the context of the invention, the terms subject or patient are to be understood as referring to a non-human subject or patient when referred to in the in vivo method steps disclosed herein.

As used herein, and unless otherwise specified, the term "tumor-bearing subject" refers to a subject that has tumor or tumor cells in its body. These tumor cells can be external to the subject and inoculated into the subject for experimentation purposes. The tumor-bearing subject can have more than one tumor. As used herein, and unless otherwise specified, the term "two-tumor model" refers to an animal model wherein the tumor-bearing animal has at least two tumors, with two tumors on distant locations. The two tumors can be contralateral to each other. The two tumors on distant locations can be of similar or same size. The two-tumor model can be used, for example, in studies related to radiation.

As used herein, and unless otherwise specified, the term "irradiated subject" refers to a subject that has received radiation. The radiation can be tumor-targeted radiation. In two-tumor model, the irradiated subject can refer to the subject that has received radiation in only one of the two distant tumors. As used herein, and unless otherwise specified, the term "non-irradiated subject" refers to a subject that has not received radiation. A "non-irradiated subject" and an "irradiated subject" can refer to the same subject before and after irradiation. A "non-irradiated subject" and an "irradiated subject" can also refer to two test subjects that are used in parallel experiments to study the effects of radiation. Such test subjects in parallel experiments can be subjects that have same genetic background, and/or have received same treatment except for radiation.

As used herein, and unless otherwise specified, the term "gene" refers to a nucleic acid molecule which is transcribed (in the case of DNA) and translated (in the case of mRNA) into a polypeptide *in vitro* or *in vivo* when placed under the control of appropriate regulatory or control sequences. The boundaries of the gene are determined by a start codon at the 5' (amino) terminus and a translation stop codon at the 3' (carboxy) terminus. A gene can include, but is not limited to, cDNA from prokaryotic or eukaryotic mRNA, genomic DNA sequences from prokaryotic or eukaryotic DNA, and even synthetic DNA sequences. A transcription termination sequence will usually be located 3' to the gene sequence.

As used herein, and unless otherwise specified, the term "candidate gene" refers to a gene that is subjected to the screening methods provided herein. The candidate gene can be knocked-out or knocked-down in order to assess the involvement of the gene in modulating the T cell immune response in combination with radiation treatment. Depending on the outcome of the screening, the "candidate gene," or the protein encoded by the "candidate gene" is identified as a suitable potential target for cancer therapy.

As used herein, and unless otherwise specified, the term "target gene" refers to a gene that can serve as a target for cancer therapy. The "target gene" can be identified from candidate genes by methods disclosed herein.

As used herein, and unless otherwise specified, the term "knock-out," when used in connection with a gene, means that the expression of the indicated gene is entirely or almost entirely abolished. The expression of the indicated gene that is knocked-out in a cell or a tissue can be reduced by at least 80%, at least 85%, at least 90%, at least 95%, at least 98%, at least 99%, or 100%, compared to its normal expression level in the cell or tissue. In some embodiments, the expression level of a gene that is knocked-out in a cell is non-detectable using ordinary means known in the art for gene expression detection.

As used herein, and unless otherwise specified, the term "knock-down," when used in connection with a gene, means that the expression of the indicated gene is significantly diminished. The expression of the indicated gene that is knocked-down in a cell or a tissue is reduced by at least 20%, at least 30%, at least 40%, at least 50%, at least 60%, at least 70%, at least 80%, or at least 90%, compared to its normal expression level in the cell or the tissue. In some embodiments, the expression level of a gene that is knocked-down in a cell or a tissue is reduced by at least 50% compared to the normal expression level of the gene in the cell or tissue.

As used herein, and unless otherwise specified, the term "isolate" refers to the process during which the indicated object is separated from at least one component present in the natural setting in which the object is normally present. The term "isolate" when used in connection with a cell or a population of cells can refer to the process of obtaining a sample containing the cell or the population of cells from the body of a subject, and/or the process of separating the cell or the population of cells from other cells or from other content in a sample containing the cell or the population of cells.

As used herein, and unless otherwise specified, the term "contact" when used in connection with a cell refers to to the process wherein the cell is placed into close proximity with the indicated substance such that the cell can interact with the substance, and/or be affected by the presence of such substance. The contact can be temporary or transient. The contact can also last a period of time or be permanent. For example, contacting a T cell with a suppressor cell can refer to the process that the T cell and the suppressor cell are cultured together in an *in vitro* container, where the cells are in such close proximity that the receptor molecules on their surfaces can interact with each other. The contact can last as long as the duration of the *in vitro* culture. Contacting a T cell with a suppressor cell can also refer to the process where the T cell is injected to a subject that has suppressor cells that would interact with the injected T cell. Such contact can be transient.

As used herein, and unless otherwise specified, the term "vector" refers to the vehicle by which a DNA or RNA sequence (*e.g*., a foreign gene) can be introduced into a host cell, so as to promote expression (*e.g*., transcription and/or translation) of the introduced sequence or to knock-down or knock-out a target gene by recombination or other mechanisms. Vectors include plasmids, phages, viruses, pseudoviruses, etc.

As used herein, and unless otherwise specified, the term "transfection" is intended to include any means, such as, but not limited to, adsorption, microinjection, electroporation, lipofection and the like, for introducing an exogenous nucleic acid molecule into a host cell.

As used herein, and unless otherwise specified, the term "T cell" refers to the type of white blood cell that completes maturation in the thymus and that has various roles in the immune system, including the identification of specific foreign antigens in the body and the activation and deactivation of other immune cells. T cells have a number of subtypes, including cytotoxic T cells, helper T cells (Th), and regulatory T cells (Treg) based on function. Differential expression of markers on the cell surface provide valuable clues to the diverse nature and function of T cells, and can serve as useful tools for the isolation of T cells or specific subtypes of T cells. For example, T cells can be isolated by selecting CD3+ cells by flow cytometry. In addition to surface markers, different subtypes of T cells can also have distinct function and cytokine secretion profiles. For example, CD8+ cytotoxic T cells destroy infected target cells through the release of perforin, granzymes, and granulysin, whereas CD4+ T helper cells have little cytotoxic activity and secrete cytokines that act on other leucocytes such as B cells, macrophages, eosinophils, or neutrophils. Tregs suppress T-cell function by several mechanisms including binding to effector T-cell subsets and preventing secretion of their cytokines.

Unless otherwise specified, a T cell can be any type of T cell and can be of any developmental stage, including but not limited to, CD4+/CD8+ double positive T cells, CD4+ helper T cells (e.g., Th1 and Th2 cells), CD8+ T cells (e.g., cytotoxic T cells), peripheral blood mononuclear cells (PBMCs), peripheral blood leukocytes (PBLs), tumor infiltrating lymphocytes (TILs), memory T cells, naïve T cells, regulator T cells, gamma delta T cells (γδ T cells), and the like. Additional types of helper T cells include cells such as Th3, Th17, Th9, or Tfh cells. Additional types of memory T cells include cells such as central memory T cells (TCM cells), effector memory T cells (TEM cells and TEMRA cells).

As used herein, and unless otherwise specified, the term "an unmodified T cell" refers to a T cell that has not been genetically modified with reference to the candidate genes being tested or screened. The unmodified T cell can be a wild type T cells. The unmodified T cell can also be a reference T cell that is genetically modified with a control gene. For example, a reference T cell can be a T cell infected with a viral vector carrying a shRNA for a control gene, such as *LacZ.* The unmodified T cell can be directly isolated from a natural source, such as a blood sample from an animal subject, or obtained by *in vitro* culturing of a line of T cells. An unmodified T cell can also be differentiated from a pluripotent cell *in vitro* by induced differentiation.

As used herein, and unless otherwise specified, the term "modified T cell" refers to a T cell that has been genetically engineered. The modified T cell can overexpress or underexpress a gene as compared to a wild type T cell. The modified T cell can have a candidate gene knocked-down or knocked out. A population of modified T cell can be a population of T cells with different genetic modification. For example, a population of modified T cell can each have different genetic signatures with at most one gene knocked-down or knocked-out as compared to a wild type T cell.

As used herein, and unless otherwise specified, the term "activity" refers to a cellular activity of the T cells. The activity can be proliferation activity, which can be enhanced cell cycle activation, suppressed cell death (e.g. suppressed apoptosis), and/or any other activities that promotes cell proliferation. The activity can also be the immunity of effector T cells, including such as cytotoxic activity or cytokine production activity. The activity can also be anti-tumor activity in general, which can be measured by, for example, tumor size reduction or increase in survival rate of a tumor bearing subject.

As used herein, and unless otherwise specified, the term "suppressor cells" refers to populations of cells that suppress T cell activity. For example, the suppressor cells can suppress T cell proliferation. The suppressor cells can also inhibit or antagonize the natural immune response elicited by T cells. "Suppressor cells" include, but not limited to, regulatory T cells (Tregs) and myeloid derived suppressor cells (MDSC).

### 4. BRIEF DESCRIPTION OF THE DRAWINGS

The following drawings form part of the present specification and are included to further demonstrate certain aspects of the present invention. The invention can be better understood by reference to one or more of these drawings in combination with the detailed description of specific embodiments presented herein.

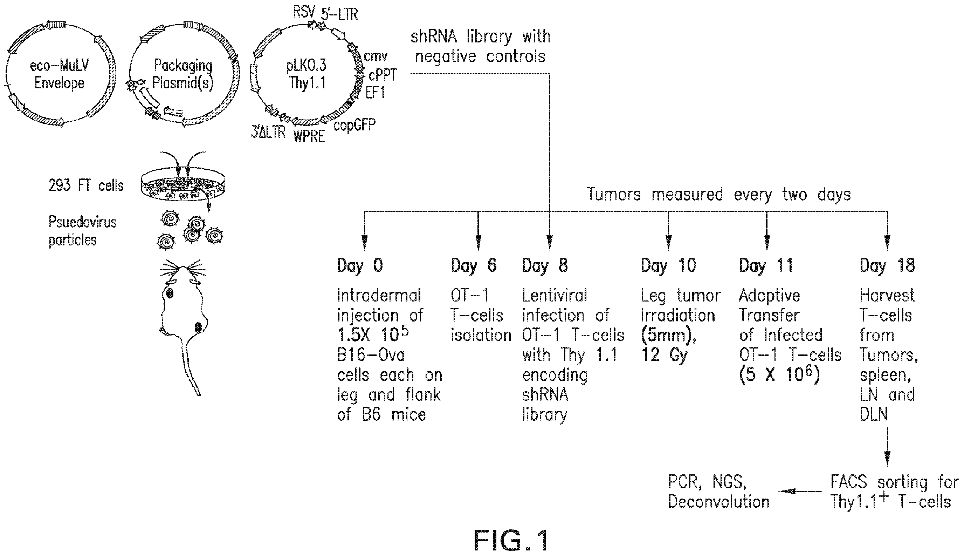

**FIG. 1** - FIG. 1 depicts an exemplary workflow for shRNA screen for immunomodulators with radiation. As shown, the workflow includes preparation of a shRNA library with negative controls; intradermal injection of 1.5 × 10⁵ B16-Ova cells each on leg and flank of B6 mice on Day 0; isolating OT-1 T cells on Day 6; infecting OT-1 T cells with Thy1.1 encoding shRNA library on Day 8; irradiating leg tumor (5mm) at 12 Gy on Day 10; performing adoptive transfer of infecting OT-1 T-cells (5×10⁶) on Day 11; harvesting T-cells from tumors from spleen, lymph nodes, and draining lymph nodes on Day 18; FACS sorting for Thy 1.1⁺ T cells; assaying genomic DNA for the Thy 1.1⁺ T cells by PCR, NGS, and data deconvoluntion. Tumor sizes are also measured every two days.

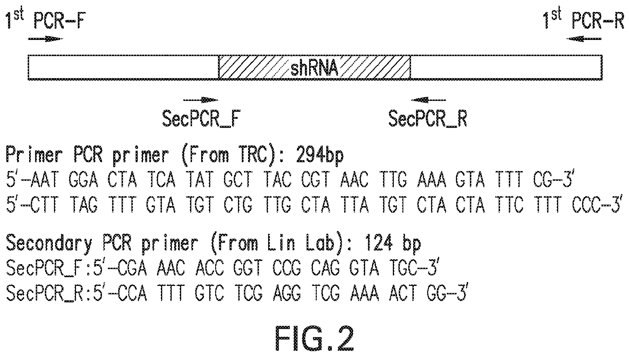

**FIG. 2** **-** FIG. 2 depicts exemplary nested primer set for PCR amplification of integrated shRNA. As shown, the primer PCR primer (294 bp) can be
   5'-AAT GGA CTA TCA TAT GCT TAC CGT AAC TTG AAA GTA TTT CG-3'
   5'-CTT TAG TTT GTA TGT CTG TTG CTA TTA TGT CTA CTA TTC TTT CCC-3';
   and the secondary PCR primer (124 bp) can be
   SecPCR_F: 5'-CGA AAC ACC GGT CCG CAG GTA TGC-3'
   SecPCR_R: 5'-CCA TTT GTC TCG AGO TCG AAA ACT GG-3'

### 5. DETAILED DESCRIPTION

Provided herein are *in vitro* and *in vivo* screening methods for identifying gene targets for cancer therapy. Without being limited by a particular theory, it is believed that a number of factors contribute to the immunosuppressive microenvironment for tumor cells, and such factors can be modulated by radiation, including but limited to tumor-targeted radiation. Thus, provided herein are *in vitro* and *in vivo* screening methods for such factors to serve as gene targets for use in cancer therapy either alone or in combination with radiation therapy. In some embodiments, provided herein is a method of identifying a target gene for cancer therapy by measuring an activity of an unmodified T cell and of a modified T cell; wherein the unmodified T cell and the modified T cell each contacted with a suppressor cell from an irradiated tumor-bearing subject; and wherein the modified T cell has a candidate gene knocked-down or knocked-out. The candidate gene is then identified as a target gene for cancer therapy if the activity is increased comparing the modified T cell to the unmodified T cell.

In some instances, radiation can place tumor cells in a "stressed" state that can lead to further activation and/or upregulation of immunosuppressive factors. Accordingly, provided herein are also screening methods for target genes that are immunosuppressive factors activated and/or upregulated by radiation treatment. Because radiation can be specifically confined to the tumor microenvironment, any perturbation within the tumor due to radiation which causes upregulation of the target of interest that interacts with T cells to induce T cell suppression can be unmasked using the screening approach described herein. The methods can further include verification of the function of the identified target genes in mediating immunosuppression.

In some instances, radiation can result in an abscopal effect, the physiological process whereby targeted radiation of a primary tumor induces an anti-tumor response a distant site outside the field of radiation. The abscopal effect, at least in part, can involve enhanced presentation of tumor antigens to T cells as well as release of cytokines and other pro-inflammatory factors that stimulate local and systemic immune responses. Accordingly, provided herein are also screening methods for gene targets involved in the abscopal effect. Gene targets identified by methods disclosed herein can be factors that contribute to the immunosuppressive nature of the tumor microenvironment.

Immunotherapy that activates the immune system, in general, to destroy cancer cells can in some instances lead to systemic toxicity due to the block of the regular checks on the immune system. Provided herein are also screening methods for factors that, when targeted alone, do not result in systematic immune activation, but can elicit significant anti-tumor immunity when used in combination with radiation therapy. Thus, agents that target these gene factors can mimic or enhance the abscopal effect, improve the therapeutic effect of radiation therapy and have reduced potential immune-related side effects.

Immunomodulatory agents for anti-tumor response in a tumor-bearing subject can, at least in part, activate the immune system by inhibiting target genes that can suppress T cell activities.

### 5.1 Screening methods

Provided herein are methods of identifying a target gene for cancer therapy by measuring an activity of an unmodified T cell and of a modified T cell; wherein the unmodified T cell and the modified T cell have each been contacted with the tumor microenvironment; and wherein the modified T cell having a candidate gene knocked-down or knocked-out; wherein the candidate gene is identified as a target gene for cancer therapy if the activity is increased if the modified T cell is compared to the unmodified T cell.

Provided herein are methods of identifying a target gene for cancer therapy by measuring an activity of an unmodified T cell and of a modified T cell; wherein the unmodified T cell and the modified T cell have each been contacted with a suppressor cell; wherein the modified T cell having a candidate gene knocked-down or knocked-out; wherein the candidate gene is identified as a target gene for cancer therapy if the activity is increased if the modified T cell is compared to the unmodified T cell.

In some embodiments, the activity measured in methods disclosed herein can be proliferation activity. In some embodiments, the proliferation activity is measured by cell cycle activation, wherein the enhanced cell cycle activation indicates an increase in cell proliferation activity. In some embodiments, the proliferation activity is measured by cell death inhibition, wherein a reduction in cell death indicates an increase in cell proliferation activity. In some embodiments, the proliferation activity is measured by apoptosis inhibition, wherein a reduction in apoptosis indicates an increase in cell proliferation activity.

Accordingly, provided herein are methods of identifying a target gene for cancer therapy by measuring the proliferation activity of an unmodified T cell and of a modified T cell; wherein the unmodified T cell and the modified T cell have each been contacted with a suppressor cell or tumor microenvironment; wherein the modified T cell having a candidate gene knocked-down or knocked-out; wherein the candidate gene is identified as a target gene for cancer therapy if the modified T cells have higher proliferation activity compared to the unmodified T cell.

In some embodiments, the activity measured in the methods disclosed herein can be the immunity of effector T cells. In some embodiments, the activity measured in methods disclosed herein is cytotoxic activity. In some embodiments, the activity measured in methods disclosed herein is cytokine production activity. In some embodiments, the activity measured in the methods disclosed herein is anti-tumor activity.

Accordingly, provided herein are methods of identifying a target gene for cancer therapy by measuring the immunity of an unmodified T cell and of a modified T cell; wherein the unmodified T cell and the modified T cell have each been contacted with a suppressor cell or tumor microenvironment; wherein the modified T cell having a candidate gene knocked-down or knocked-out; wherein the candidate gene is identified as a target gene for cancer therapy if the modified T cells have higher immunity compared to the unmodified T cell.

Provided herein are methods of identifying a target gene for cancer therapy by measuring the cytotoxic activity of an unmodified T cell and of a modified T cell; wherein the unmodified T cell and the modified T cell have each been contacted with a suppressor cell or tumor microenvironment; wherein the modified T cell having a candidate gene knocked-down or knocked-out; wherein the candidate gene is identified as a target gene for cancer therapy if the modified T cells have higher cytotoxic activity compared to the unmodified T cell.

Also provided herein are methods of identifying a target gene for cancer therapy by measuring the cytokine production activity of an unmodified T cell and of a modified T cell; wherein the unmodified T cell and the modified T cell have each been contacted with a suppressor cell or tumor microenvironment; wherein the modified T cell having a candidate gene knocked-down or knocked-out; wherein the candidate gene is identified as a target gene for cancer therapy if the modified T cells have higher cytokine production activity compared to the unmodified T cell.

Also provided herein are methods of identifying a target gene for cancer therapy by measuring the anti-tumor activity of an unmodified T cell and of a modified T cell; wherein the unmodified T cell and the modified T cell have each been contacted with a suppressor cell or tumor microenvironment; wherein the modified T cell having a candidate gene knocked-down or knocked-out; wherein the candidate gene is identified as a target gene for cancer therapy if the modified T cells have higher anti-tumor activity compared to the unmodified T cell.

In some embodiments, the methods described herein further include obtaining T cells from a source. The methods provided herein can further include preparing unmodified T cells and modified T cells. In some embodiments, the source is a sample from a subject. In some embodiments, the source is a cell line cultured *in vitro.*

In some embodiments, the methods described herein include measuring the activity of the unmodified T cell and of the modified T cell each having been contacted with the tumor microenvironment from both an irradiated tumor-bearing subject and a non-irradiated tumor-bearing subject; wherein the candidate gene is identified as target gene if the increase in activity comparing the modified T cell to the unmodified T cell is more significant when T cells have been contacted with the tumor microenvironment of an irradiated tumor-bearing subject than when T cells have been with the tumor microenvironment of a non-irradiated tumor-bearing subject. In some embodiments, the activity of the unmodified T cell and modified T cell each having been contacted with the tumor microenvironment of a non-irradiated tumor-bearing subject are substantially the same.

In some embodiments, the methods described herein include using a first population of suppressor cells from a non-irradiated tumor-bearing subject, and a second population of suppressor cells from an irradiated tumor-bearing subject. The methods can include measuring the increase in the activity comparing modified T cells to unmodified T cells each have been contacted with the first population of suppressor cells, and also measuring the increase in the activity comparing modified T cells to unmodified T cells each have been contacted with the second population of suppressor cells; wherein the candidate gene is identified as target gene if the increase is more significant when T cells have been contacted with second population of suppressor cells than when T cells have been contacted with first population of suppressor cells. In some embodiments, the activity is substantially the same comparing the modified T cell and the unmodified T cell when T cells have been contacted with first population of suppressor cells, but higher in the modified T cell when the T cells have been contacted with the second population of suppressor cells.

In some embodiments, the T cells used in methods described herein are isolated form a sample directly obtained from an animal subject. The sample can be a blood sample. The blood sample can be a whole blood sample, a partially purified blood sample, or a peripheral blood sample. The sample can also be a bone marrow sample. T cells can be isolated from a sample by its surface markers. In some embodiments, the T cells used in methods described herein are CD3+ cells. In some embodiments, the T cells used in methods described herein are CD8+ cells. In some embodiments, the T cells are CD4+ cells. In some embodiments, the T cells are naive T cells. In some embodiments, the T cells are naïve CD8+ T cells. In some embodiments, the T cells naive CD4+ T cells. In some embodiments, the T cells are CD4+/CD8+ double positive T cells. In some embodiments, the T cells are PBMCs. In some embodiments, the T cells are PBLs. In some embodiments, the T cells are TILs. In some embodiments, the T cells are memory T cells. In some embodiments, the T cells are gamma delta T cells. In some embodiments, the T cells are as Th3, Th17, Th9, or Tfh cells. In some embodiments, the T cells are TCM cells, TEM cells or TEMRA cells. The T cells can also be any combination of the specific types of T cells mentioned herein or otherwise known in the art.

In some embodiments, the T cells are isolated from a subject. The subject can be a mammal. In some embodiments, the T cells are isolated form a mouse. The mouse can be a wild type mice or a genetically engineered mouse. In some embodiments, the mouse is genetically engineered to expresses a T-cell receptor. The T-cell receptor can be specific for an antigen that is not naturally present in mice. In some embodiments, the antigen can be ovalbumine. In some embodiments, the subject can be a mouse genetically engineered to express a T-cell receptor specific for ovalbumin.

In some embodiments, provided herein are methods of identifying a target gene for cancer therapy by measuring an activity of an unmodified CD8+ T cell and of a modified CD8+ T cell; the unmodified CD8+ T cell and the modified CD8+ T cell each having been contacted with a suppressor cell; wherein the modified CD8+ T cell having a candidate gene knocked-down or knocked-out; wherein the candidate gene is identified as a target gene for cancer therapy if the activity is increased in the modified CD8+ T cell compared to the unmodified CD8+ T cell.

In some embodiments, the T cells are produced by induced differentiation from a stem cell or progenitor cell. The stem cell or progenitor cell can be a hematopoietic stem cell or a hematopoietic progenitor cell. The stem cell or progenitor cell can also be an induced pluripotent stem cell.

The unmodified T cells can be genetically modified using methods described herein or otherwise known in the art to produce modified T cells. In some embodiments, the T cells are modified to have a candidate gene knocked-out or knocked-down by infection with a viral vector that contains shRNA. In some embodiments, the unmodified T cells are reference T cells with a control gene knocked-out or knocked-down. In one embodiment, the viral vector is a vector from lentivirus. In some embodiments, the viral vector is SMARTvector, GIPZ, TRIPZ or TRC lentiviral shRNA vector (GE Dharmacon). In some embodiments, the gene in the isolated T cells is knocked-out or knocked-down by using a transcription activator-like effector nuclease (TALEN). In some embodiments, the gene in the isolated T cells is knocked-out or knocked-down by using CAS9 (CRISPR). As a person of ordinary skill in the art would understand, any methods for genetic modulation of T cells described herein or otherwise known in the art can be used in the screening methods described herein.

In some embodiments, the unmodified T cells are expanded *in vitro* before being subjected to genetic modification or the screening methods described herein. In some embodiments, the modified T cells are expanded *in vitro* before being subjected to the screening methods described herein.

In one embodiment, T cells used in methods described herein are labeled. In some embodiments, T cells are labeled with a fluorescent agent. In some embodiments, T cells are labeled with a micron size particle. In some embodiments, T cells are labeled with carboxyfluorescein succinimidyl ester (CFSE). In some embodiments, T cells are labeled with micron-sized iron oxide particles (MPIQs). In some embodiments, T cells are labeled magnetic nanoparticles. A variety of methods for labeling T cells are well known in the art.

The candidate gene can be any gene naturally present in the genome of a T cell. The candidate gene can be a human gene. The candidate gene can also be a gene from a model animal. For example, the candidate gene can also be a gene of mouse T cells.

In some embodiments, screening methods provided herein include preparing a starting population of T cells includes infecting unmodified T cells with a shRNA library. In some embodiments, the shRNA library is a genomic library, which includes viral vectors that can screen all genes of a T cell genome. In some embodiments, the shRNA library is a subgenomic library, which includes a selection of genes of a T cell genome. In some embodiments, the shRNA library includes viral vectors that target genes that encode certain proteins with particular structural/functional features. Exemplary libraries include, but are not limited to, G-protein coupled receptor (GPCR) library, tumor necrosis factor receptor (TNFR) superfamily library, immunoglobulin superfamily (IgSF) library, transcription factor library, kinase library, phosphatase library, angiogenesis library, cell cycle library, apoptosis library, and/or ubiquitin-ligase library. Exemplary libraries also can be focused on genes known to be overexpressed or underexpressed in dysfunctional T cells. For example, the library of candidate genes can be a library of genes overexpressed in T-cell anergy or exhaustion. Libraries used in methods disclosed herein can also be any combination of the libraries described herein or otherwise known in the art.

In some embodiments, the suppressor cells include Treg cells. In some embodiments, the suppressor cells include MDSCs. In some embodiments, the suppressor cells include both Treg cells and MSDCs. In some embodiments, wherein the screening methods are carried out *in vitro*, the suppressor cells can be isolated from a subject. The animal can be a mammal. In some embodiments, the suppressor cells can be isolated from a mouse. In some embodiments, the subject can be an irradiated subject. In some embodiments, the subject can be a non-irradiated subject. In some embodiments, the suppressor cells are from an irradiated mouse. In some embodiments, the suppressor cells are from a non-irradiated mouse. In some embodiments, the mouse can be a tumor-bearing mouse. The mouse can be a non-irradiated tumor-bearing mouse. The mouse can be an irradiated tumor-bearing mouse. In some embodiments, the subject is a mouse having at least two tumors wherein only one tumor has received an ablative dose of radiation

In some embodiments, the suppressor cells are isolated from a blood sample. The blood sample can be a whole blood sample, a partially purified blood sample, or a peripheral blood sample. Methods to obtain isolated suppressor cells from a sample for T cell suppression assays are well known in the art. In some embodiments, wherein the screening methods are carried out *in vivo,* the suppressor cells can be the naturally present suppressor cells in the test subject, which can also include Tregs, MSDCs, or both. The subject can be an irradiated subject or a non-irradiated subject.

In some embodiments, the suppressor cells are from a non-irradiated tumor-bearing subject. In some embodiments, the suppressor cells are from an irradiated tumor-bearing subject. In some embodiments, the irradiated tumor-bearing subject has received tumor-targeted radiation. In some embodiments, the radiation has been given at an ablative dose. In some embodiments, the radiation has been given at a dose of from 1 Gy to 45 Gy, 5 Gy to 35 Gy, 10 Gy to 25 Gy, 1 Gy to 25 Gy, 1 Gy to 20 Gy, or 5 Gy to 15 Gy. In some embodiments, the radiation has been given at a dose of from 1 Gy to 20 Gy. In some embodiments, the radiation has been given at a dose of 1 Gy, 2 Gy, 3 Gy, 4 Gy, 5 Gy, 6 Gy, 7 Gy, 8 Gy, 9 Gy, 10 Gy, 11 Gy, 12 Gy, 13 Gy, 14 Gy, 15 Gy, 16 Gy, 17 Gy, 18 Gy, 19 Gy, 20 Gy, 25 Gy, 30 Gy, 35 Gy, 40 Gy, or 45 Gy. In some embodiments, the suppressor cells are from a tumor-bearing subject that has received an ablative dose of radiation targeted at its primary tumor.

In some embodiments, the tumor-bearing subject has one tumor. In some embodiments, the tumor-bearing subject has two or more tumors. In some embodiments, the tumor-bearing subject is a two-tumor model. In some embodiments, the irradiated tumor-bearing subject having two or more tumors has received radiation targeted at only one of its tumors. The tumor that receives radiation can be the primary tumor of the tumor-bearing subject.

In some embodiments, the subject can be a mouse. The mouse can be a wild type mouse or a genetically engineered mouse. In some embodiments, the mouse is genetically engineered to expresses a T-cell receptor. The T-cell receptor can be specific for an antigen that is not naturally present in mice. In some embodiments, the antigen can be ovalbumine. In some embodiments, the subject can be a mouse genetically engineered to express a T-cell receptor specific for ovalbumin. The mouse can be a tumor-bearing mouse. The mouse can be a non-irradiated tumor-bearing mouse. The mouse can be an irradiated tumor-bearing mouse. In some embodiments, the subject is a mouse having at least two tumors wherein one tumor has received an ablative dose of radiation.

The screening methods provided herein include *in vitro* methods and *in vivo* methods. As a person of ordinary skill in the art would understand, different *in vitro* and *in vivo* methods described herein can be used either separately or in combination. Gene targets identified by a combination of more than one method can potentially be more complete and more accurate. Additionally, the methods described herein can be pool screening assays in high throughput format. For example, an *in vitro* screening test provided herein can be conducted first to identify a group of candidate genes to serve as targets for cancer therapeutics, followed by an *in vivo* functional test to further corroborate their utility.

### 5.1.3 In vivo Assays

In some embodiments, described herein are *in vivo* methods for screening a target gene for cancer therapy. The *in vivo* assay can be performed on non-human test subjects. The test subject can be an animal model. The animal model can be a two-tumor model. In some embodiments, the animal model is a mouse. In some embodiments, the animal model is a tumor-bearing animal model. The animal model can be a tumor-bearing mouse. The tumor-bearing mouse can be irradiated or non-irradiated. In some embodiments, the animal model is a mouse having two tumors at contralateral positions, only one of which has received an ablative dose of radiation.

Accordingly, provided herein are *in vivo* methods of identifying a target gene for cancer therapy including (i) injecting unmodified T cell and modified T cells to test subjects, the modified T cell having a candidate gene knocked-down or knocked-out; and (ii) measuring an activity of the unmodified T cell and of the modified T cell in the test subjects, wherein the candidate gene is identified as a target gene for cancer therapy if the activity is increased comparing the modified T cell to the unmodified T cell. The test subject can be an irradiated test subject. The test subject can be a non-irradiated test subject. In some embodiments, the irradiated test subject receives radiation before T cell injection. In some embodiments, the irradiated test subject receives radiation after T cell injection but before the activity is measured. In some embodiments, the unmodified T cells and modified T cells are injected into the same test subject. In some embodiments, the unmodified T cells and modified T cells are injected to separate test subjects. In some embodiments, the T cells used in methods described herein are labeled. In some embodiments, the unmodified T cells are reference T cells.

In some embodiments, the irradiated tumor-bearing subject has received tumor-targeted radiation. In some embodiments, the radiation has been given at an ablative dose. In some has been embodiments, the radiation has been given at a dose of from 1 Gy to 45 Gy, 5 Gy to 35 Gy, 10 Gy to 25 Gy, 1 Gy to 25 Gy, 1 Gy to 20 Gy, or 5 Gy to 15 Gy. In some embodiments, the radiation has been given at a dose of from 1 Gy to 20 Gy. In some embodiments, the radiation has been given at a dose of 1 Gy, 2 Gy, 3 Gy, 4 Gy, 5 Gy, 6 Gy, 7 Gy, 8 Gy, 9 Gy, 10 Gy, 11 Gy, 12 Gy, 13 Gy, 14 Gy, 15 Gy, 16 Gy, 17 Gy, 18 Gy, 19 Gy, 20 Gy, 25 Gy, 30 Gy, 35 Gy, 40 Gy, or 45 Gy. In some embodiments, the tumor-bearing subject is a two tumor model that has received an ablative dose of radiation targeted at only one of its tumors.

The T cells used in *in vivo* screening methods disclosed herein can be any specific type of T cells methods described herein or otherwise known in the art. For example, in some embodiments, the T cells used in methods described herein are CD3+ cells. In some embodiments, the T cells used in methods described herein are CD8+ cells. In some embodiments, the T cells are CD4+ cells. In some embodiments, the T cells are naive T cells. In some embodiments, the T cells are naive CD8+ T cells. In some embodiments, the T cells naïve CD4+ T cells.

In some embodiments, the measuring step includes obtaining a sample from the test subject. The sample can be a tumor sample, a tissue sample, or a blood sample. The tissue sample can be a lymph node sample, a spleen sample, a liver sample, or a kidney sample. The lymph node sample can be a draining lymph node or a non-draining lymph node. The tumor sample can be a sample from an irradiated tumor. The tumor sample can also be a sample from a non-irradiated tumor. The non-irradiated tumor can be from a test subject that has an irradiated tumor.

In some embodiments, the *in vivo* screening methods described herein include using non-irradiated subjects and irradiated subjects. The methods can include measuring the increase in the activity comparing modified T cells to unmodified T cells injected to non-irradiated subjects, and also measuring the increase in the activity comparing modified T cells to unmodified T cells injected to irradiated subjects; wherein the candidate gene is identified as target gene if the increase is more significant when T cells are injected to irradiated subjects than when T cells are injected to non-irradiated subjects. In some embodiments, the activities of the T cells are substantially the same comparing the modified T cell and the unmodified T cell when T cells are injected to non-irradiated subjects, but higher in the modified T cell compared to the unmodified T cells when T cells are injected to irradiated subjects.

In some embodiments, the activity measured by *in vivo* methods described herein can be proliferation activity of the T cells. In some embodiments, the activity measured by *in vivo* methods described herein can be anti-tumor activity of the T cells. The proliferation activity of T cells can be measured by the accumulation of T cells injected into the test subject post injection. The accumulation of T cells can measured 24 hours post injection, 2 days post injection, 3 days post injection, 4 days post injection, 5 days post injection, 6 days post injection, 7 days post injection, 8 days post injection, 9 days post injection, 10 days post injection, 11 days post injection, 12 days post injection, 13 days post injection, 14 days post injection, 2.5 weeks post injection, 3 weeks post injection, 3.5 weeks post injection, 4 weeks post injection, 5 weeks post injection, 6 weeks post injection, 7 weeks post injection, 2 months post injection, 3 months post injection, or longer.

The accumulation of T cells in the subject can be measured by a variety of approaches known in the art. In some embodiments, the accumulation of injected T cells in the test subject can be measured by cell cytometry. In some embodiments, the accumulation of T cells in the test subject can be measured by next generation sequencing (NGS) of the genomic DNA from the accumulated T cells.

Accordingly, provided herein are *in vivo* methods of identifying a target gene for cancer therapy including (i) injecting unmodified T cell and modified T cells to test subjects, the modified T cell having a candidate gene knocked-down or knocked-out; and (ii) measuring the accumulation of the unmodified T cell and of the modified T cell in the test subjects, wherein the candidate gene is identified as a target gene for cancer therapy if a larger number of modified T cell are accumulated as compared to the unmodified T cell. In some embodiments, the unmodified T cells are reference T cells. The test subject can be a mouse. The test subject can be tumor-bearing. The test subject can be an irradiated tumor-bearing test subject. In some embodiments, the irradiated test subject receives radiation before T cell injection. In some embodiments, the irradiated test subject receives radiation after T cell injection but before the activity is measured. In some embodiments, the unmodified T cells and modified T cells are injected in to the same test subject.

In some embodiments, provided herein are *in vivo* methods of identifying a target gene for cancer therapy including (i) injecting unmodified T cell and modified T cells to test subjects, the modified T cell having a candidate gene knocked-down or knocked-out; and (ii) measuring the number of tumor-infiltrating unmodified T cell and the number of tumor-infiltrating modified T cell in the test subjects, wherein the candidate gene is identified as a target gene for cancer therapy if the number of tumor-infiltrating modified T cell is higher as compared to the number of tumor-infiltrating unmodified T cell. In some embodiments, the unmodified T cells are reference T cells. The test subject can be a tumor-bearing mouse. The test subject can be an irradiated tumor-bearing test subject. In some embodiments, the irradiated test subject receives radiation before T cell injection. In some embodiments, the irradiated test subject receives radiation after T cell injection but before the activity is measured. In some embodiments, the unmodified T cells and modified T cells are injected in to the same test subject.

In some embodiments, the methods provided herein further include isolated T cells accumulated/proliferated from the injected T cells from the test subjects. This isolation step can include obtaining a sample from the test subjects. The sample can be a tumor sample, a blood sample, or a tissue sample. The tumor sample can be a sample from an irradiated tumor or a non-irradiated tumor. The non-irradiated tumor can be from a test subject having an irradiated tumor. The blood sample can be a sample of the peripheral blood, the whole blood, or partially purified blood. The tissue sample can be a spleen sample, or a lymph node sample. The lymph node sample can be a draining lymph node sample or a non-draining lymph node sample.

In some embodiments, the activity measured by *in vivo* methods described herein can be anti-tumor activity of the injected T cells. The anti-tumor activity can be measured by the reduction of tumor size over a period of time. The period of time can be 24 hours, 2 days, 3 days, 4 days, 5 days, 6 days, 7 days, 8 days, 9 days, 10 days, 11 days, 12 days, 13 days, 14 days, 2.5 weeks, 3 weeks, 3.5 weeks, 4 weeks, 5 weeks, 6 weeks, 7 weeks, 2 months, 3 months, or longer.

The anti-tumor activity can also be measured by the survival rate of a population of test subjects. Accordingly, provided herein are *in vivo* methods of identifying a target gene for cancer therapy including (i) injecting unmodified T cell and modified T cells to separate test subjects, the modified T cell having a candidate gene knocked-down or knocked-out; and (ii) measuring the tumor size of the subject injected with unmodified T cell and of the subject injected with modified T cell, wherein the candidate gene is identified as a target gene for cancer therapy if a reduction in tumor size is greater in the test subject injected with modified T cells as compared to that in test subject injected with unmodified T cell. In some embodiments, the unmodified T cells are reference T cells. The test subject can be a tumor-bearing mouse. The test subject can be an irradiated tumor-bearing test subject. In some embodiments, the irradiated test subject receives radiation before T cell injection. In some embodiments, the irradiated test subject receives radiation after T cell injection but before the tumor size is measured. In some embodiments, the test subjects are two-tumor models, wherein only one tumor received targeted radiation. In some embodiment, the size of the irradiated tumor is measured. In some embodiments, the size of a non-irradiated tumor is measured.

Also provided herein are *in vivo* methods of identifying a target gene for cancer therapy including (i) injecting unmodified T cell and modified T cells separately to a population of tumor-bearing test subjects, the modified T cell having a candidate gene knocked-down or knocked-out; and (ii) measuring the survival rate of the population of tumor-bearing subjects injected with unmodified T cell and survival rate of the population of tumor-bearing subjects injected with modified T cell, wherein the candidate gene is identified as a target gene for cancer therapy if a greater survival rate is achieved in the population of subjects injected with modified T cells as compared to the population of subjects injected with unmodified T cell. In some embodiments, the unmodified T cells are reference T cells. The test subjects can be an irradiated test subjects. In some embodiments, the irradiated test subjects receive radiation before T cell injection. In some embodiments, the irradiated test subject receives radiation after T cell injection. In some embodiments, the test subjects are two-tumor models, wherein only one tumor received targeted radiation. In some embodiments, the test subjects are mice.

In some embodiments, provided herein is a method of identifying a target for cancer therapy comprising:
(1) preparing a set of T cells from a subject, wherein a candidate gene is selectively knocked-out or knocked-down in the isolated T cells;
(2) implanting two tumors into each of a group of test subjects, wherein the two tumors are implanted distally;
(3) providing a first group of test subjects, wherein tumors have not been treated with radiation, and a second group of test subjects, wherein only one of the tumors has been treated with radiation;
(4) transferring the T cells into a subject from the first group and assessing reduction of tumor; and
(5) transferring the T cells into a subject from the second group and assessing reduction of the tumor that has not been treated with radiation;
wherein the gene knocked-out or knocked-down in the T cells, or the protein encoded thereby, is identified as a target for cancer therapy if reduction of tumor observed in step (5) is higher than the reduction observed in step (4).

In one embodiment, the subject is a mouse. In another embodiment, the mouse is a transgenic mouse. In another embodiment, the mouse is a transgenic mouse engineered to amplify T cell immune response by producing a monoclonal population of antigen-specific T cells.

In one embodiment, the tumor in the second group of test subjects has been treated by radiation at a dose of from 1 Gy to 45 Gy, 5 Gy to 35 Gy, 10 Gy to 25 Gy, 1 Gy to 25 Gy, 1 Gy to 20 Gy, or 5 Gy to 15 Gy. In one embodiment, the dose is from 1 Gy to 20 Gy.

In one embodiment, the gene in the isolated T cells is knocked-out or knocked-down by infecting the T cells with a viral vector that contains shRNA. In one embodiment, the viral vector is a vector from lentivirus. In another embodiment, the gene in the isolated T cells is knocked-out or knocked-down by using a transcription activator-like effector nuclease (TALEN). In another embodiment, the gene in the isolated T cells is knocked-out or knocked-down by using CAS9 (CRISPR).

### 5.1.2 In vitro Assay

In some embodiments, described herein are *in vitro* methods for screening a target gene for cancer therapy. The *in vitro* assay can be a T cell suppression assay. The *in vitro* assay can include culturing T cells with suppressor cells. In some embodiments, the methods provided herein further include isolating suppressor cells from a subject. The subject can be an irradiated subject or a non-irradiated subject.

Accordingly, in some embodiments, provided herein are *in vitro* methods for screening a target gene for cancer therapy including culturing separately (a) unmodified T cells and suppressor cells; and (b) modified T cells and suppressor cells; wherein the modified T cells have a candidate gene knocked-down or knocked-out; and measuring an activity of the unmodified T cells and of the modified T cells, wherein the candidate gene is identified as a target gene for cancer therapy if the activity is increased comparing the modified T cell to the unmodified T cell. In some embodiments, the unmodified T cells are reference T cells. The methods can include providing isolated suppressor cells. The suppressor cells can be isolated from a sample from a subject. The subject can be a mouse. The sample can be a blood sample. The suppressor cells can be isolated based on its surface marker.

The T cells and suppressor cells can be cultured for a period of time. The period of time can be 30 minutes, 1 hour, 2 hours, 3 hours, 4 hours, 5 hours, 6 hours, 7 hours, 8 hours, 9 hours, 10 hours, 12 hours, 14 hours, 16 hours, 18 hours, 20 hours, 22 hours, 24 hours, 2 days, 3 days, 4 days, 5 days, 6 days, 7 days, or longer.

In some embodiments, T cells are activated with anti-CD3 and anti-CD28 in the presence of suppressor cells, at various T cell versus suppressor cell ratios. In some embodiments, T cells are activated with anti-CD3 and anti-CD28. In some embodiments, the methods include culturing T cells and suppressor cells at about 10:1, 5:1, 2:1, 1:1, 1:2, or 1:5 ratio. In some embodiments, the methods include culturing T cells and suppressor cells at about 1:1 ratio.

In one embodiment, T cells used in methods described herein are labeled. In some embodiments, T cells are labeled with a fluorescent agent. In some embodiments, T cells are labeled with a micron size particle. In some embodiments, T cells are labeled with carboxyfluorescein succinimidyl ester (CFSE). In some embodiments, T cells are labeled with micron-sized iron oxide particles (MPIOs). In some embodiments, T cells are labeled magnetic nanoparticles. A variety of methods for labeling T cells are well known in the art.

The *in vitro* screening methods can measure activities including, but not limited to proliferation, cell cycle activation, cell death, cytokine production, and/or cytotoxic activity.

Suppression of T cells by suppressor cells (*e*.*g*., Tregs and MDSCs) provided herein can be assayed using various methods well known in the art. Such methods include, but are not limited to, those described in Dolcetti et al., Current Protocols of Immunology, 14.17.1-14.17.25 (2010); Bayne et al., Cold Spring Harb Protoc, doi: 10.1101/pdb.prot077214 (2013); Kruisbeek et al, Current Protocols in Immunology, 3.12.1-3.12.20 (2004); and Collison et al., Methods Mol Biol. 707: 21-37 (2011).

In some embodiments, the suppressor cells include Treg cells. In some embodiments, the suppressor cells include MDSCs. In some embodiments, the suppressor cells include both Treg cells and MSDCs. In some embodiments, wherein the screening methods are carried out *in vitro*, the suppressor cells can be isolated from a subject. The animal can be a mammal. In some embodiments, the suppressor cells can be isolated from a mouse. In some embodiments, the subject can be an irradiated subject. In some embodiments, the subject can be a non-irradiated subject. In some embodiments, the suppressor cells are from an irradiated mouse. In some embodiments, the suppressor cells are from a non-irradiated mouse.

In some embodiments, the *in vitro* screening methods described herein include using a first population of suppressor cells isolated from a non-irradiated tumor-bearing subject, and a second population of suppressor cells isolated from an irradiated tumor-bearing subject. The methods can include measuring the increase in the activity comparing modified T cells to unmodified T cells each cultured with the first population of suppressor cells, and also measuring the increase in the activity comparing modified T cells to unmodified T cells each cultured with the second population of suppressor cells; wherein the candidate gene is identified as target gene if the increase is more significant when T cells are cultured with second population of suppressor cells than when T cells are cultured with first population of suppressor cells. In some embodiments, the activities of the T cells are substantially the same comparing the modified T cell and the unmodified T cell when T cells are cultured with first population of suppressor cells, but higher in the modified T cell compared to the unmodified T cells when the T cells are cultured with the second population of suppressor cells.

In some embodiments, provided herein are *in vitro* methods for screening a target gene for cancer therapy by testing cell cycle activation of T cells. The method can include culturing separately (a) unmodified T cells and suppressor cells; and (b) modified T cells and suppressor cells; wherein the modified T cells having a candidate gene knocked-down or knocked-out; and measuring the cell cycle activation of the unmodified T cells and of the modified T cells, wherein the candidate gene is identified as a target gene for cancer therapy if the cell cycle activation is enhanced in the modified T cell compared to the unmodified T cell. In some embodiments, the cell cycle activation is measured by incorporation of 3[H]-thymidine incorporation in to the DNA of T cells. In some embodiments, the unmodified T cells are reference T cells. In some embodiments, the methods including activating T cells with anti-CD3 and anti-CD28.

In some embodiments, provided herein are *in vitro* methods for screening a target gene for cancer therapy by testing cell death of T cells. In some embodiments, the methods including activating T cells with anti-CD3 and anti-CD28. The method can include culturing separately (a) unmodified T cells and suppressor cells; and (b) modified T cells and suppressor cells; wherein the modified T cells having a candidate gene knocked-down or knocked-out; and measuring the cell death of the unmodified T cells and of the modified T cells, wherein the candidate gene is identified as a target gene for cancer therapy if cell death is reduced in the modified T cell compared to the unmodified T cell. In some embodiments, the unmodified T cells are reference T cells.

The cell death can be apoptosis, mitotic cell death, and necrosis. In some embodiments, methods provided herein measure apoptosis. In some embodiments, methods provided herein measure apoptosis mitotic cell death. In some embodiments, methods provided herein measure necrosis. A variety of assay to measure cell death are known in the art, with kits commercially available. In some embodiments, cell death is measured by a caspase assay. In some embodiments, cell death is measured by DNA fragmentation or strand breaks. In some embodiments, cell death is measured by cell cytometry. In some embodiments cell death is measured by ELISA.

In other embodiments, provided herein are methods of assessing the development of T cell effector function in response to antigen-specific activation. Assessing the cytotoxic activity of T cells, elicited by antigen-specific stimulation *in vitro,* allows one to assess the functional cytolytic potential of effector cells. In some embodiments, one can assess either alloantigen stimulation or antigen-specific triggering, with minor modifications. In certain embodiments, evaluation of cytolytic activity by 51Cr release, can be used to investigate T cell-specific effector function and its modulation/inhibition by immunomodulatory cells, while preserving the simplicity of the assay and allowing the investigator to work with numerous variables and rare cell populations. Chromium release assay can provide a more specific functional readout.

In certain embodiments, lytic unit transformation, which measures the extent of suppression normalized to the internal control without suppressive cells, provides a more useful representation of the results and allows comparing and averaging of results from different experiments. Evaluation of L.U. represents a more effective measure than either the proliferation assay or single effector-to- target ratio cytotoxicity value, since it includes an estimation of both functional activity (cytolytic activity of cultures) and cell proliferation (number of cells recovered in each culture).

In some embodiments, the antigen is a protein that is not naturally present in the target cells. In one embodiment, the antigen is ovalbumin.

In some embodiments, provided herein are *in vitro* methods for screening a target gene for cancer therapy by testing cytokine production of T cells. In some embodiments, the methods including activating T cells with anti-CD3 and anti-CD28. The method can include culturing separately (a) unmodified T cells and suppressor cells; and (b) modified T cells and suppressor cells; wherein the modified T cells have a candidate gene knocked-down or knocked-out; and measuring the cytokine production of the unmodified T cells and of the modified T cells, wherein the candidate gene is identified as a target gene for cancer therapy if the cytokine production is enhanced in the modified T cell compared to the unmodified T cell. In some embodiments, the cytokine measured in methods described herein include interferon (IFN)-γ, tumor necrosis factor (TNF)-α, interleukin (IL)-2, Granulocyte-macrophage colony-stimulating factor (GM-CSF), or any combination thereof. A variety of methods to test cytokine production are known in the art. For example, cytokines can be measure by an immunoblotting (IB) assay, an immunofluorescent (IF) assay, FACS, ELISA, or Intracellular cytokine staining (ICS). Kits for testing cytokine production are also commercially available.

In one embodiment, provided herein is a method of identifying a target for cancer therapy comprising:
(1) providing a first set of T cells from a subject, wherein no modification is made in the isolated T cells, and a second set of T cells, wherein a candidate gene is selectively knocked-out or knocked-down in the isolated T cells;
(2) contacting the first set of T cells with suppressor cells isolated from a tumor-bearing subject, wherein the tumor has not been treated by radiation and determining the extent of T cell suppression in the sample;
(3) contacting the first set of T cells with suppressor cells isolated from a tumor-bearing subject, wherein the tumor has been treated by radiation and determining the extent of T cell suppression in the sample;
(4) contacting the second set of T cells with suppressor cells isolated from a tumor-bearing subject, wherein the tumor has been treated by radiation and
   determining the extent of T cell suppression in the sample;
(5) determining the reduction in T cell suppression in step (4) as compared to step (3);
(6) determining the reduction in T cell suppression in step (4) as compared to step (3); and
(7) comparing the difference in reduction as determined in step (5) to the difference in reduction as determined in step (6);
wherein the gene knocked-out or knocked-down in the T cells, or the protein encoded thereby, is identified as a target for cancer therapy if the difference in reduction of T cell suppression as determined in step (6) is larger than the reduction as determined in step (5).

In one embodiment, the subject is a mouse.

In another embodiment, the mouse is genetically engineered to have T cell receptor with particular antigen specificity. In another embodiment, the antigen is a protein not naturally present in mice. In another embodiment, the antigen is ovalbumin.

In one embodiment, the T cells are labeled. In another embodiment, the T cells are labeled with carboxyfluorescein succinimidyl ester (CFSE).

In one embodiment, the tumor has been treated with an ablative dose of radiation. In another embodiment, the tumor has been treated by radiation at a dose of from 1 Gy to 45 Gy, 5 Gy to 35 Gy, 10 Gy to 25 Gy, 1 Gy to 25 Gy, 1 Gy to 20 Gy, or 5 Gy to 15 Gy. In one embodiment, the tumor has been treated by radiation at a dose of from 1 Gy to 20 Gy.

In one embodiment, the candidate gene in the isolated T cells is knocked-out or knocked-down by infecting the T cells with a viral vector that contains shRNA. In one embodiment, the viral vector is a vector from lentivirus. In some embodiments, the viral vector is SMART vector, GIPZ, TRIPZ or TRC lentiviral shRNA vector (GE Dharmacon). In another embodiment, the gene in the isolated T cells is knocked-out or knocked-down by using a transcription activator-like effector nuclease (TALEN). In another embodiment, the gene in the isolated T cells is knocked-out or knocked-down by using CAS9 (CRISPR).

In one embodiment, T cell suppression is assessed by monitoring proliferation of T cells. In one embodiment, proliferation of T cells is assessed in the presence of anti-CD3 + anti-CD28. In one embodiment, the proliferation of T cells is determined by incorporation of ³[H]-thymidine. In another embodiment, the proliferation is determined by flowcytometric analysis of T cells labeled with carboxyfluorescein succinimidyl ester (CFSE).

In one embodiment, T cell suppression is assessed by mesuraing the cytotoxic activity of T cells. In one embodiment, the cytotoxic activity is elicited by antigen-specific stimulation. In one embodiment, the antigen is ovalbumin.

In one embodiment, the method is conducted in arrayed format in a high throughput setting.

In certain embodiments, provided herein is a method of identifying a target for cancer therapy comprising:
(1) co-culturing T cells from a subject with suppressor cells isolated from a tumor-bearing subject, wherein the tumor has not been treated by radiation, in a first cell culture:
(2) co-culturing the T cells with suppressor cells from a tumor-bearing subject, wherein the tumor has been treated by radiation, in a second cell culture;
(3) infecting the first and second cultures with viral vectors designed to introduce a series of knockdown or knockout of candidate genes and further culturing the cells; and
(4) determining the relative levels of particular gene constructs in the cultures;
wherein a gene construct that is determined to be represented at a higher level in the second culture as compared to the first culture, or the protein encoded thereby, is identified to be a target.

In one embodiment, the viral vectors are lentivirus vectors.

In another embodiment, the relative levels of particular gene constructs are determined by sequencing the vectors. In one embodiment, the sequencing is next generation sequencing.

A person of ordinary skill in the art would understand that the screening methods provided herein can include measuring one or more types of T cell activity with any combination of *in vitro* assays described herein or otherwise known in the art.

### 5.1.3 Pool Assays

Provided herein are also high throughput pool screening methods for identifying a target gene for cancer therapy. The pool screen methods can include i) preparing a starting population of T cells with different genetic modifications, wherein each T cell has one candidate gene knocked-down or knocked-out; and (ii) contacting the starting population of T cells with the tumor microenvironment for a period of time to produce a selected population of T cells; wherein the candidate gene that is knocked-down or knocked-out in the selected population of T cells is identified to be the target gene for cancer therapy. In some embodiments, each T cell in the starting population has at most one candidate gene knocked-down or knocked-out.

Provided herein are also high throughput pool screening methods for identifying a target gene for cancer therapy. The pool screen methods can include i) preparing a starting population of T cells with different genetic modifications, wherein each T cell has one candidate gene knocked-down or knocked-out; and (ii) contacting the starting population of T cells with suppressor cells for a period of time to produce a selected population of T cells; wherein the candidate gene that is knocked-down or knocked-out in the selected population of T cells is identified to be the target gene for cancer therapy. In some embodiments, each T cell in the starting population has at most one candidate gene knocked-down or knocked-out.

The T cells used in pool screening methods disclosed herein can be any specific type of T cells methods described herein or otherwise known in the art. For example, in some embodiments, the T cells used in methods described herein are CD3+ cells. In some embodiments, the T cells used in methods described herein are CD8+ cells. In some embodiments, the T cells are CD4+ cells. In some embodiments, the T cells are naive T cells. In some embodiments, the T cells are naive CD8+ T cells. In some embodiments, the T cells naive CD4+ T cells.

In some embodiments, the T cells are isolated from a subject. The subject can be a mammal. In some embodiments, the T cells are isolated form a mouse. The mouse can be a wild type mice or a genetically engineered mouse. In some embodiments, the mouse is genetically engineered to expresses a T-cell receptor. The T-cell receptor can be specific for an antigen that is not naturally present in mice. In some embodiments, the antigen can be ovalbumine. In some embodiments, the subject can be a mouse genetically engineered to express a T-cell receptor specific for ovalbumin.

In one embodiment, T cells used in methods described herein are labeled. In some embodiments, T cells are labeled with a fluorescent agent. In some embodiments, T cells are labeled with a micron size particle. In some embodiments, T cells are labeled with carboxyfluorescein succinimidyl ester (CFSE). In some embodiments, T cells are labeled with micron-sized iron oxide particles (MPIOs). In some embodiments, T cells are labeled magnetic nanoparticles. A variety of methods for labeling T cells are well known in the art.

The selected population of T cells results from the proliferation the starting population of T cells. T cells with genetic modifications that increase the proliferation of the T cells are accumulated in the selected population of T cells. Thus, the candidate gene that is knocked-down or knocked-out in the selected population of T cells indicates that targeting these genes can enhance T cell proliferation in a tumor-bearing subject. In some embodiments, the tumor-bearing subject can be an irradiated tumor-bearing subject.

In some embodiments, the candidate gene that is knocked-down or knocked-out in the selected population of T cells is identified by NGS of genomic DNAs isolated from the selected population of T cells.

In some embodiments, preparing a starting population of T cells includes infecting unmodified T cells with a shRNA library. In some embodiments, the shRNA library is a genomic library, which includes viral vectors that can screen all genes of a T cell genome. In some embodiments, the shRNA library is a subgenomic library, which includes a selection of genes of a T cell genome. In some embodiments, the shRNA library includes viral vectors that target genes that encode certain proteins with particular structural/functional features. Exemplary libraries include, but are not limited to, G-protein coupled receptor (GPCR) library, tumor necrosis factor receptor (TNFR) superfamily library, immunoglobulin superfamily (IgSF) library, transcription factor library, kinase library, phosphatase library, angiogensis library, cell cycle library, apoptpsis library, and/or ubiquitin-ligase library. Exemplary libraries also can be focused on genes known to be overexpressed or underexpressed in dysfunctional T cells. For example, the library of candidate genes can be a library of genes overexpressed in T-cell anergy or exhaustion. Libraries used in methods disclosed herein can also be any combination of the libraries described herein or otherwise known in the art.

In some embodiments, the shRNA library can include control shRNA, such as *LacZ* shRNA.

In some embodiments, preparing a starting population of T cells includes using a transcription activator-like effector nuclease (TALEN). In some embodiments, preparing a starting population of T cells includes using CAS9 (CRISPR). In some embodiments, preparing a starting population of T cells includes infecting T cells with a shRNA library. In some embodiments, the shRNA library is a lentivirus vector library.

In some embodiments, the pool screening methods described herein can be an *in vitro* method wherein the contacting step includes *in vitro* culturing the starting T cell population with suppressor cells. In some embodiments, the suppressor cells can be isolated from an irradiated tumor-bearing subject. In some embodiments, the suppressor cells can be isolated from a non-irradiated tumor-bearing subject.

In some embodiments, T cells are activated with anti-CD3 and anti-CD28 in the presence of suppressor cells, at various T cell versus suppressor cell ratios. In some embodiments, T cells are activated with anti-CD3 and anti-CD28. In some embodiments, the methods include culturing T cells and suppressor cells at about 10:1, 5:1, 2:1, 1:1, 1:2, or 1:5 ratio. In some embodiments, the methods include culturing T cells and suppressor cells at 1:1 ratio.

In some embodiments, the suppressor cells are from an irradiated tumor-bearing subject. In some embodiments, the suppressor cells are from a non-irradiated tumor-bearing subject.

Accordingly, provided herein are *in vitro* high throughput pool screening methods for identifying a target gene for cancer therapy. The *in vitro* pool screen methods can include i) preparing a starting population of T cells with different genetic modifications, wherein each T cell has a candidate gene knocked-down or knocked-out; and (ii) culturing the starting population of T cells with suppressor cells for a period of time to produce a selected population of T cells; wherein the candidate gene that is knocked-down or knocked-out in the selected population of T cells is identified to be the target gene for cancer therapy. In some embodiments, each T cell in the starting population has at most one candidate gene knocked-down or knocked-out. In some embodiments, the methods further include isolating suppressor cells from an irradiated tumor-bearing subject. In some embodiments, the methods further include isolating suppressor cells from a non-irradiated tumor-bearing subject.

In some embodiments, the period of time for culturing the T cells and the suppressor cells can be 24 hours, 2 days, 3 days, 4 days, 5 days, 6 days, 7 days, 8 days, 9 days, 10 days, 11 days, 12 days, 13 days, 14 days, 2.5 weeks, 3 weeks, 3.5 weeks, 4 weeks, 5 weeks, 6 weeks, 7 weeks, 2 months, 3 months, or longer.

As a person of ordinary skill in the art would understand, any permutations of the *in vitro* screening methods described in the section above also apply to these *in vitro* pool screening methods.

In some embodiments, the pool screening methods described herein can be an *in vivo* method wherein the contacting step includes injecting the starting T cells population into a tumor-bearing non-human test subject. Accordingly, provided herein are *in vivo* high throughput pool screening methods for identifying a target gene for cancer therapy. The *in vivo* pool screen methods can include i) preparing a starting population of T cells with different genetic modifications, wherein each T cell has at most one candidate gene knocked-down or knocked-out; and (ii) injecting the starting population of T cells with suppressor cells to a tumor-bearing test subject to produce a selected population of T cells; wherein the candidate gene that is knocked-down or knocked-out in the selected population of T cells is identified to be the target gene for cancer therapy.

The test subject can be a non-human animal model. The animal model can be a two-tumor model. In some embodiments, the animal model is a mouse. The tumor-bearing mouse can be irradiated or non-irradiated. In some embodiments, the animal model is a two-tumor model. In some embodiments, the animal model is a mouse having two tumors at coutralateral positions, only one of which has received an ablative dose of radiation.

The methods can also include isolating the selected pool of T cells from the subject post injection. The selected pool of T cells can be isolated from a sample of the test subject. The sample can be a tumor sample, a blood sample, or a tissue sample. The tumor sample can be a sample from an irradiated tumor or a non-irradiated tumor. The blood sample can be a sample of the peripheral blood, the whole blood, or partially purified blood. The tissue sample can be a spleen sample, or a lymph node sample. The lymph node sample can be a draining lymph node sample or a non-draining lymph node sample.

In some embodiments, the methods comprising isolating the selected population of T cells from the test subject 24 hours post injection, 2 days post injection, 3 days post injection, 4 days post injection, 5 days post injection, 6 days post injection, 7 days post injection, 8 days post injection, 9 days post injection, 10 days post injection, 11 days post injection, 12 days post injection, 13 days post injection, 14 days post injection, 2.5 weeks post injection, 3 weeks post injection, 3.5 weeks post injection, 4 weeks post injection, 5 weeks post injection, 6 weeks post injection, 7 weeks post injection, 2 months post injection, 3 months post injection, or longer.

The selected population of T cells in the subject can be measured by a variety of approaches known in the art. In some embodiments, the accumulation of injected T cells in the test subject can be measured by cell cytometry. In some embodiments, the accumulation of T cells in the test subject can be measured by next generation sequencing (NGS) of the genomic DNA from the accumulated T cells.

In some embodiments, each T cell of the starting population has at most a shRNA for a candidate gene, and a candidate gene is identified to be a target gene for cancer therapy if the shRNA for the candidate gene is enriched in the selected population of T cells as compared to the starting population of T cells. T cells with these target genes knocked-down or knocked-out can have enhanced proliferation independent of TCR recognition of a tumor antigen. In some embodiments, the candidate gene is enriched in the selected population of T cells as compared to the starting population of T cells by about 2 fold, about 3 fold, about 4 fold, about 5 fold, about 6 fold, about 7 fold, about 8 fold, about 9 fold, about 10 fold, about 12 fold, about 15 fold, about 20 fold, about 25 fold, about 30 fold, about 35 fold, about 40 fold, about 45 fold, or about 50 fold. In some embodiments, the candidate gene is enriched in the selected population of T cells as compared to the starting population of T cells by at least 2 fold, at least 3 fold, at least 4 fold, at least 5 fold, at least 6 fold, at least 7 fold, at least 8 fold, at least 9 fold, at least 10 fold, at least 12 fold, at least 15 fold, at least 20 fold, at least 25 fold, at least 30 fold, at least 35 fold, at least 40 fold, at least 45 fold, at least 50 fold, or more.

shRNA that restores T cells proliferation in the tumors can be enriched in tumor but not other tissues, such as a second lymphoid organ (e.g. spleen). Accordingly, in some embodiments, a candidate gene is identified to be a target gene for cancer therapy if the shRNA for the candidate gene is enriched in the selected population of T cells obtained from a tumor sample as compared to a non-tumor tissue sample. The tumor sample can be a sample from an irradiated tumor or a non-irradiated tumor. The tissue sample can be a second lymphoid organ sample, such as a spleen sample, or a lymph node sample. The lymph node sample can be a draining lymph node sample or a non-draining lymph node sample.

In some embodiments, the candidate gene is enriched in the tumor sample compared to the non-tumor tissue sample by about 2 fold, about 3 fold, about 4 fold, about 5 fold, about 6 fold, about 7 fold, about 8 fold, about 9 fold, about 10 fold, about 12 fold, about 15 fold, about 20 fold, about 25 fold, about 30 fold, about 35 fold, about 40 fold, about 45 fold, or about 50 fold. In some embodiments, the candidate gene is enriched in the selected population of T cells as compared to the starting population of T cells by at least 2 fold, at least 3 fold, at least 4 fold, at least 5 fold, at least 6 fold, at least 7 fold, at least 8 fold, at least 9 fold, at least 10 fold, at least 12 fold, at least 15 fold, at least 20 fold, at least 25 fold, at least 30 fold, at least 35 fold, at least 40 fold, at least 45 fold, at least 50 fold, or more.

In some embodiments, quantifying shRNA in the selected population of T cells can include using NGS.

As a person of ordinary skill in the art would understand, any permutations of the *in vivo* screening methods described in the section above also apply to these *in vivo* pool screening methods.

As a person of ordinary skill in the art would understand, the pool screening methods described herein can also include further functional testing to corroborate the target genes identified therein.

### 5.2 Knock-Out or Knock-Down of Candidate Genes in T Cells

In some embodiments, a candidate gene is knocked-out or knocked-down in T cells in order to assess the effect of the absence of a particular protein in regulating T cell function in combination with radiation. Any conventional methods known in the art for gene editing may be employed in connection with methods provided herein.

### 5.2.1 RNAi Based Knock-Out or Knock-Down

Knock-out or knock-down (collectively "silencing") of a candidate gene may be achieved in a variety of cell systems using chemically synthesized or *in vitro* transcribed small interfering RNA (siRNA), as well as PCR or DNA vector-based short hairpin RNA (shRNA). A few promoters have been reported to drive shRNA expression in cells, including RNA polymerase III-based promoters, U6 and H1, and RNA polymerase II promoter, CMV. In addition, the shRNA molecules provided herein may be operably linked to a T cell-specific promoter to achieve T cell specific targeting of the shRNA molecules to achieve silencing of the candidate genes.

In certain embodiments, provided herein are methods of gene silencing by RNA interference with a candidate gene. The method involves creating constructs that encode the interfering (silencing) RNA in which a promoter that is active in the cell type to which the construct is going to be delivered (*e.g*., T cells) is used to drive the expression of the shRNA.

In the shRNA constructs used in the methods provided herein, there are small stretches of nucleotides that are directed against the candidate gene and are for use in modulating the expression of nucleic acid molecules encoding the candidate gene. Inhibition of a candidate gene is accomplished by providing oligomeric compounds which hybridize with one or more target nucleic acid molecules encoding the candidate gene.

The principle behind antisense technology is that an antisense compound, which hybridizes to a target nucleic acid, modulates gene expression activities such as transcription or translation. This sequence specificity makes antisense compounds extremely attractive as tools for target validation and gene functionalization.

Without being limited by a particular theory, the mechanism of action for the silencing of a candidate gene involves the hybridization of the shRNA compounds with the target nucleic acid, wherein the outcome or effect of the hybridization is either target degradation or target occupancy with concomitant stalling of the cellular machinery involving, for example, transcription or splicing of the candidate gene.

Examples of an occupancy-based antisense mechanism whereby antisense compounds hybridize yet do not elicit cleavage of the target include, but are not limited to, inhibition of translation, modulation of splicing, modulation of poly(A) site selection and disruption of regulatory RNA structure. A method of controlling the behavior of a cell through modulation of the processing of an mRNA target by contacting the cell with an antisense compound acting via a non-cleavage event is described, for example, in U.S. Pat. No. 6,210,892 and U.S. Publication No. 20020049173.

Generally, the sense sequence of the shRNA will be from about 19 to about 22 nucleotides (*e*.*g*., about 19, 20, 21 or 22 nucleotides) in length, the antisense sequence will be from about 19 to about 22 nucleotides (*e.g*., about 19, 20, 21 or 22 nucleotides), in length, and the loop region will be from about 3 to about 19 nucleotides (*e.g*., from about 3 up to about 19) nucleotides in length. In some embodiments, the sense and antisense sequences are the same length, *i*.*e*., the shRNA will form a symmetrical hairpin. In other embodiments, the sense or antisense strand may be shorter than its complementary strand, and an asymmetric hairpin may be formed. In some embodiments, the base pairing between the sense and antisense sequences is exact. In other embodiments, some mismatch between the sequences may be tolerated, or even desired, for example, to decrease the strength of the hydrogen bonding between the two strands. In some embodiments, the shRNA molecule can also comprise a 5'-terminal phosphate group that can be chemically modified. In addition, the loop portion of the shRNA molecule can comprise, for example, nucleotides, non-nucleotides, linker molecules, conjugate molecules and the like.

Any suitable vectors can be used in connection with the RNAi silencing of a candidate gene. Examples include, but are not limited to: virus-based vectors such as adenoviral, lentiviral, adeno-associated viral, and retroviral vectors; and plasmid-based vectors and other vectors such as baculovirus, phage, phagemids, cosmids, phosmids, bacterial artificial chromosomes, PI-based artificial chromosomes, yeast plasmids, and yeast artificial chromosomes.

In one embodiment, the vector is a viral vector. In another embodiment, the viral vector is lentivirus vector.

Delivery of shRNA expressing vectors can be by administration to target cells or by any other means that would allow for introduction into the desired target cell(s) or tissue, for example, transfection or transduction.

### 5.2.2 TALEN Based Knock-Out or Knock-Down

In other embodiments, a candidate gene can be silenced using a transcription activator-like effector and nuclease (TALEN) based system. Transcription activator-like (TAL) effector sequences can be assembled to bind DNA targets with specificity by assembling sequences of repeat variable-diresidue (RVDs). Fusion proteins of TAL effectors and nucleases (TALENs) can make targeted double-stranded breaks in cellular DNA that can be used to make specific genetic modifications to cells. TALENs have been reported as useful to generate stably modified cells.

The TAL effector domain that binds to a specific nucleotide sequence within the target DNA can comprise 10 or more DNA binding repeats, or 15 or more DNA binding repeats. Each DNA binding repeat can include an RVD that determines recognition of a base pair in the target DNA sequence, wherein each DNA binding repeat is responsible for recognizing one base pair in the target DNA sequence, and wherein the RVD comprises one or more of: HD for recognizing C; NG for recognizing T; NI for recognizing A; NN for recognizing G or A; NS for recognizing A or C or G or T; N* for recognizing C or T, where * represents a gap in the second position of the RVD; HG for recognizing T; H* for recognizing T, where * represents a gap in the second position of the RVD; IG for recognizing T; NK for recognizing G; HA for recognizing C; ND for recognizing C; HI for recognizing C; HN for recognizing G; NA for recognizing G; SN for recognizing G or A; and YG for recognizing T.

In some embodiments, a TALEN can comprise an endonuclease domain and a TAL effector DNA binding domain specific for a target DNA, wherein the DNA binding domain comprises a plurality of DNA binding repeats, each repeat comprising a RVD that determines recognition of a base pair in the target DNA, wherein each DNA binding repeat is responsible for recognizing one base pair in the target DNA, and wherein the TALEN comprises one or more of the following RVDs: HD for recognizing C; NG for recognizing T; NI for recognizing A; NN for recognizing G or A; NS for recognizing A or C or G or T; N* for recognizing C or T; HG for recognizing T; H* for recognizing T; IG for recognizing T; NK for recognizing G; HA for recognizing C; ND for recognizing C; HI for recognizing C; HN for recognizing G; NA for recognizing G; SN for recognizing G or A; and YG for recognizing T. The TALEN can comprise one or more of the following RVDs: HA for recognizing C; ND for recognizing C; HI for recognizing C; HN for recognizing G; NA for recognizing G; SN for recognizing G or A; YG for recognizing T; and NK for recognizing G, and one or more of: HD for recognizing C; NG for recognizing T; NI for recognizing A; NN for recognizing G or A; NS for recognizing A or C or G or T; N* for recognizing C or T; HG for recognizing T; H* for recognizing T; and IG for recognizing T. The endonuclease domain can be from a type II restriction endonuclease (*e*.*g*., FokI).

In some embodiments, a cell can be transiently transformed, such that the construct is not integrated into its genome. For example, a plasmid vector containing a TALEN coding sequence can be introduced into a cell, such that the TALEN coding sequence is expressed but the vector is not stably integrated in the genome. Transiently transformed cells typically lose some or all of the introduced nucleic acid construct with each cell division, such that the introduced nucleic acid cannot be detected in daughter cells after sufficient number of cell divisions. However, expression of the TALEN coding sequence can be sufficient to achieve homologous recombination between a donor sequence and an endogenous target sequence. Both transiently transformed and stably transformed cells can be useful in the methods provided herein.

The polynucleotides and/or recombinant vectors can be introduced into the genome of a host using any conventional methods known in the art, including, but not limited to, electroporation, microinjection, and biolistic methods. In addition, any other gene transfer and transformation techniques conventionally known in the art can be used in connection with methods provided herein. Such techniques include, but are not limited to, protoplast transformation through calcium or PEG, electroporation-mediated uptake of naked DNA, liposome-mediated transfection, electroporation, viral vector-mediated transformation, and microprojectile bombardment (*see* U.S. Pat. Nos. 5,538,880, 5,204,253, 5,591,616, and 6,329,571). In some embodiments, a DNA modifying enzyme (*e.g*., a TALEN) can be directly introduced into a cell. For example, a polypeptide can be introduced into a cell by mechanical injection, by delivery via a bacterial type III secretion system, or by electroporation.

In some embodiments, the nucleic acid binding is linked, for example, with an effector domain that includes but is not limited to a transposase, integrase, recombinase, resolvase, invertase, protease, DNA methyltransferase, DNA demethylase, histone acetylase, histone deacetylase, nuclease, transcriptional repressor, transcriptional activator, transcription factor recruiting, protein nuclear-localization signal or cellular uptake signal.

In some embodiments, the effector domain is a protein domain which exhibits activities which include but are not limited to transposase activity, integrase activity, recombinase activity, resolvase activity, invertase activity, protease activity, DNA methyltransferase activity, DNA demethylase activity, histone acetylase activity, histone deacetylase activity, nuclease activity, nuclear-localization signaling activity, transcriptional repressor activity, transcriptional activator activity, transcription factor recruiting activity, or cellular uptake signaling activity. Other embodiments may include any combination the activities described herein.

Any suitable vectors, including cloning and expression vectors, as well as viral vectors and integrating vectors, can be used in connection with methods provided herein. An "expression vector" is a vector that includes one or more expression control sequences, and an "expression control sequence" is a DNA sequence that controls and regulates the transcription and/or translation of another DNA sequence. Suitable expression vectors include, but are not limited to, plasmids and viral vectors derived from, for example, bacteriophage, baculoviruses, tobacco mosaic virus, herpes viruses, cytomegalovirus, retroviruses, vaccinia viruses, adenoviruses, and adeno-associated viruses. Numerous vectors and expression systems are commercially available. By way of example, some vectors used in recombinant DNA techniques allow entities, such as a segment of DNA (such as a heterologous DNA segment, such as a heterologous cDNA segment), to be transferred into a target cell.

Vectors may have one or more restriction endonuclease recognition sites (whether type I, II or IIs) at which the sequences may be cut in a determinable fashion without loss of an essential biological function of the vector, and into which a nucleic acid fragment may be spliced or inserted in order to bring about its replication and cloning. Vectors may also comprise one or more recombination sites that permit exchange of nucleic acid sequences between two nucleic acid molecules. Vectors may further provide primer sites, *e*.*g*., for PCR, transcriptional and/or translational initiation and/or regulation sites, recombinational signals, replicons, and selectable markers. Vectors may further contain one or more selectable markers suitable for use in the identification of cells transformed with the vector.

In some embodiments, where translation of the desired nucleic acid sequence is required, for example, the mRNA encoding a TALE polypeptide, the vector also may comprise sequences required for proper translation of the nucleotide sequence.

In some embodiments, expression vectors are often in the form of "plasmids," which refer to circular double-stranded DNA loops which, in their vector form, are not bound to a chromosome. In some embodiments, all components of a given TALE polypeptide may be encoded in a single vector. For example, in some embodiments, a vector may be constructed that contains or may comprise all components necessary for a functional TALE polypeptide. In some embodiments, individual components (*e.g*., one or more monomer units and one or more effector domains) may be separately encoded in different vectors and introduced into one or more cells separately. Moreover, any vector described herein may itself comprise predetermined TALE polypeptide encoding component sequences, such as an effector domain and/or TALE monomer unit, at any location or combination of locations.

Examples of vectors include, but are not limited to, plasmids, episomes, bacteriophages, or viral vectors, and such vectors may integrate into a host cell's genome or replicate autonomously in the particular cellular system used. In some embodiments, the vector used is an episomal vector, *i*.*e*., a nucleic acid capable of extra-chromosomal replication and may include sequences from bacteria, viruses or phages. In other embodiments, vectors are derived from bacterial plasmids, bacteriophages, yeast episomes, yeast chromosomal elements, and viruses, vectors derived from combinations thereof, such as those derived from plasmid and bacteriophage genetic elements, cosmids and phagemids. In some embodiments, a vector may be a plasmid, bacteriophage, bacterial artificial chromosome (BAC) or yeast artificial chromosome (YAC). A vector may be a single- or double-stranded DNA, RNA, or phage vector.

Viral vectors include, but are not limited to, retroviral vectors, such as lentiviral vectors or gammaretroviral vectors, adenoviral vectors, and baculoviral vectors. For example, a lentiviral vector may be used in the form of lentiviral particles. Other forms of expression vectors known by those skilled in the art which serve equivalent functions may also be used. Expression vectors may be used for stable or transient expression of the polypeptide encoded by the nucleic acid sequence being expressed. A vector may be a self-replicating extrachromosomal vector or a vector which integrates into a host genome. In one embodiment, vector is a genomic integrated vector, or "integrated vector," which may become integrated into the chromosomal DNA or RNA of a host cell, cellular system, or non-cellular system. In some embodiments, the nucleic acid sequence encoding the TALE polypeptides or component sequences integrates into the chromosomal DNA or RNA of a host cell, cellular system, or non-cellular system along with components of the vector sequence.

The recombinant expression vectors provided herein may comprise a TALE nucleic acid in a form suitable for expression of the nucleic acid in a host cell, which indicates that the recombinant expression vector(s) include one or more regulatory sequences, selected on the basis of the host cell(s) to be used for expression, which is operatively linked to the nucleic acid sequence to be expressed.

### 5.2.3 CRISPR/CAS9 Based Knock-Out or Knock-Down

In some embodiments, a candidate gene is silenced using Clustered Regularly Interspaced Short Palindromic Repeats (CRISPR)/CAS9 system described, for example, in Wiedenheft et al., Nature 482 (7385): 331-8 (2012), the entirety of which is incorporated herein by reference.

Briefly, an engineered, programmable, non-naturally occurring CRISPR-Cas system comprising a Cas9 protein and one or more guide RNAs that target the genomic loci of DNA molecules encoding one or more gene products in a eukaryotic cell is provided, and the Cas9 protein cleaves the genomic loci of the DNA molecules encoding the one or more gene products, altering expression of the one or more gene products. (*See* U.S. Patent No. 8,697,359).

Vectors can be designed for expression of CRISPR transcripts (e.g. nucleic acid transcripts, proteins, or enzymes) in prokaryotic or eukaryotic cells. For example, CRISPR transcripts can be expressed in bacterial cells such as Escherichia coli, insect cells (using baculovirus expression vectors), yeast cells, or mammalian cells. Suitable host cells are discussed further in Goeddel, GENE EXPRESSION TECHNOLOGY: METHODS IN ENZYMOLOGY 185, Academic Press, San Diego, Calif. (1990). Alternatively, the recombinant expression vector can be transcribed and translated in vitro, for example using T7 promoter regulatory sequences and T7 polymerase.

Vectors may be introduced and propagated in a prokaryote. In some embodiments, a prokaryote is used to amplify copies of a vector to be introduced into a eukaryotic cell or as an intermediate vector in the production of a vector to be introduced into a eukaryotic cell (*e*.*g*., amplifying a plasmid as part of a viral vector packaging system). In some embodiments, a prokaryote is used to amplify copies of a vector and express one or more nucleic acids, such as to provide a source of one or more proteins for delivery to a host cell or host organism.

In some embodiments, the vectors are fusion expression vectors. Examples of such vectors include, but are not limited to, commercially available vectors such as pGEX (Pharmacia Biotech Inc), pMAL (New England Biolabs) and pRIT5 (Pharmacia, Piscataway, N.J.) that fuse glutathione S-transferase (GST), maltose E binding protein, or protein A. respectively, to the target recombinant protein. Examples of suitable inducible non-fusion E. coli expression vectors include, but are not limited to, pTrc (Amrann et al., Gene 69:301-315 (1988)) and pET 11d (Studier et al., GENE EXPRESSION TECHNOLOGY: METHODS IN ENZYMOLOGY 185, Academic Press, San Diego, Calif. 60-89 (1990)).

In some embodiments, a vector is a yeast expression vector. Examples of such vectors include, but are not limited to, pYepSec1 (Baldari et al., EMBO J. 6: 229-234 (1987)), pMFa (Kuijan and Herskowitz, 1982. Cell 30: 933-943), pJRY88 (Schultz et al., Gene 54: 113-123 (1987)), pYES2 (Invitrogen Corporation, San Diego, Calif), and picZ (Invitrogen Corp, San Diego, Calif).

In some embodiments, vectors are mammalian expression vectors. Examples such vectors include, but are not limited to, pCDM8 (Seed, Nature 329: 840 (1987)) and pMT2PC (Kaufman et al., EMBO J. 6: 187-195 (1987)). When used in mammalian cells, the expression vector's control functions are typically provided by one or more regulatory elements. For example, commonly used promoters are derived from polyoma, adenovirus 2, cytomegalovirus, simian virus 40, and others known in the art.

In some embodiments, a regulatory element is operably linked to one or more elements of a CRISPR system so as to drive expression of the one or more elements of the CRISPR system. CRISPR loci have been identified in more than 40 prokaryotes including, but not limited to, *Aeropyrum*, *Pyrobaculum*, *Sulfolobus*, *Archaeoglobus*, *Halocarcula*, *Methanobacteriumn*, *Methanococcus*, *Methanosarcina*, *Methanopyrus*, *Pyrococcus*, *Picrophilus*, *Thernioplasnia*, *Corynebacterium*, *Mycobacterium*, *Streptomyces*, *Aquifrx*, *Porphvromonas*, *Chlorobium*, *Thermus*, *Bacillus*, *Listeria*, *Staphylococcus*, *Clostridium*, *Thermoanaerobacter*, *Mycoplasma*, *Fusobacterium*, *Azarcus*, *Chromobacterium*, *Neisseria*, *Nitrosomonas*, *Desulfovibrio*, *Geobacter*, *Myrococcus*, *Campylobacter*, *Wolinella*, *Acinetobacter*, *Erwinia*, *Escherichia*, *Legionella*, *Methylococcus*, *Pasteurella*, *Photobacterium, Salmonella*, *Xanthomonas*, *Yersinia*, *Treponema*, and *Thermotoga*.

In some embodiments, one or more elements of a CRISPR system is derived from a type I, type II, or type III CRISPR system. In some embodiments, one or more elements of a CRISPR system is derived from a particular organism comprising an endogenous CRISPR system, such as *Streptococcus pyogenes.*

Typically, a CRISPR system is characterized by elements that promote the formation of a CRISPR complex at the site of a target sequence (also referred to as a protospacer in the context of an endogenous CRISPR system). The term "target sequence," when used in connection with CRISPR system, refers to a sequence to which a guide sequence is designed to have complementarity, where hybridization between a target sequence and a guide sequence promotes the formation of a CRISPR complex. In some embodiments, full complementarity may not be necessary, provided there is sufficient complementarity to cause hybridization and promote formation of a CRISPR complex.

A target sequence may comprise any polynucleotide, such as DNA or RNA polynucleotides. In some embodiments, a target sequence is located in the nucleus or cytoplasm of a cell. In some embodiments, the target sequence is located within an organelle of a eukaryotic cell, for example, mitochondrion or chloroplast.

In some embodiments, a vector comprises one or more insertion sites, such as a restriction endonuclease recognition sequence (also referred to as a "cloning site"). In some embodiments, one or more insertion sites (e.g. about or more than about 1, 2, 3, 4, 5, 6, 7, 8, 9, 10, or more insertion sites) are located upstream and/or downstream of one or more sequence elements of one or more vectors. In some embodiments, a vector comprises an insertion site upstream of a tracr mate sequence, and optionally downstream of a regulatory element operably linked to the tracr mate sequence, such that following insertion of a guide sequence into the insertion site and upon expression the guide sequence directs sequence-specific binding of a CRISPR complex to a target sequence in a eukaryotic cell. In some embodiments, a vector comprises two or more insertion sites, each insertion site being located between two tracr mate sequences so as to allow insertion of a guide sequence at each site. In such an arrangement, the two or more guide sequences may comprise two or more copies of a single guide sequence, two or more different guide sequences, or combinations of these. When multiple different guide sequences are used, a single expression construct may be used to target CRISPR activity to multiple different, corresponding target sequences within a cell. For example, a single vector may comprise about or more than about 1, 2, 3, 4, 5, 6, 7, 8, 9, 10, 15, 20, or more guide sequences. In some embodiments, about or more than about 1, 2, 3, 4, 5, 6, 7, 8, 9, 10, or more such guide-sequence-containing vectors may be provided, and optionally delivered to a cell.

In some embodiments, a vector comprises a regulatory element operably linked to an enzyme-coding sequence encoding a CRISPR enzyme, such as a Cas protein. Examples of Cas proteins include, but are not limited to, Cas1, Cas1B, Cas2, Cas3, Cas4, Cas5, Cas6, Cas7, Cas8, Cas9 (also known as Csn1 and Csx12), Cas10, Csy1, Csy2, Csy3, Cse1, Cse2, Csc1, Csc2, Csa5, Csn2, Csm2, Csm3, Csm4, Csm5, Csm6, Cmr1, Cmr3, Cmr4, Cmr5, Cmr6, Csb1, Csb2, Csb3, Csx17, Csx14, Csx10, Csx16, CsaX, Csx3, Csx1, Csx15, Csf1, Csf2, Csf3, Csf4, and homologs or derivatives thereof.

In some embodiments the CRISPR enzyme is Cas9, and may be Cas9. In some embodiments, the CRISPR enzyme directs cleavage of one or both strands at the location of a target sequence, such as within the target sequence and/or within the complement of the target sequence. In some embodiments, the CRISPR enzyme directs cleavage of one or both strands within about 1, 2, 3, 4, 5, 6, 7, 8, 9, 10, 15, 20, 25, 50, 100, 200, 500, or more base pairs from the first or last nucleotide of a target sequence. In some embodiments, a vector encodes a CRISPR enzyme that is mutated to with respect to a corresponding wild-type enzyme such that the mutated CRISPR enzyme lacks the ability to cleave one or both strands of a target polynucleotide containing a target sequence.

In some embodiments, an enzyme coding sequence encoding a CRISPR enzyme is codon optimized for expression in particular cells, such as eukaryotic cells. The eukaryotic cells may be those of or derived from a particular organism, such as a mammal, including but not limited to human, mouse, rat, rabbit, dog, or non-human primate. Codon optimization refers to a process of modifying a nucleic acid sequence for enhanced expression in the host cells of interest by replacing at least one codon of the native sequence with codons that are more frequently or most frequently used in the genes of that host cell while maintaining the native amino acid sequence.

Various species exhibit particular bias for certain codons of a particular amino acid. Codon bias (differences in codon usage between organisms) often correlates with the efficiency of translation of messenger RNA (mRNA), which is in turn believed to be dependent on, among other things, the properties of the codons being translated and the availability of particular transfer RNA (tRNA) molecules. The predominance of selected tRNAs in a cell is generally a reflection of the codons used most frequently in peptide synthesis. Accordingly, genes can be tailored for optimal gene expression in a given organism based on codon optimization.

A guide sequence may be selected to target any target sequence. In some embodiments, the target sequence is a sequence within a genome of a cell. Exemplary target sequences include those that are unique in the target genome. For example, for the S. pyogenes Cas9, a unique target sequence in a genome may include a Cas9 target site of the form MMMMMMMMNNNNNNNNNNNNXG where NNNNNNNNNNNNXGG (N is A, G, T, or C; and X can be anything) has a single occurrence in the genome.

Several commercially available CRISPR/Cas9 systems for gene editing are available in the art. Any such systems, and any variations thereof, can be used in connection with methods provided herein.

### 5.3 Methods of use

Also provided herein are methods of use of the gene target identified with the screening methods described herein. For example, provided herein are methods for activating a T cell by inhibiting the target gene identified by the screening methods described herein. In some embodiments, T cell is present in a tumor-bearing subject. The tumor-bearing subject can receive tumor targeted radiation before, concurrently, or after the treatment that inhibits the target gene identified by the screening methods described herein.

Disclosed herein are also methods to treat cancer in a subject in need thereof by inhibiting the target gene identified by screening methods described herein. In some embodiments, provided herein are also methods to treat cancer in a subject in need thereof by administering a T cell therapy using T cells having target gene identified by screening methods described herein knocked-down or knocked-out. In some instances, the methods can further include administering a tumor-targeting radiation therapy.

### 6. EXAMPLES

Embodiments provided herein may be more fully understood by reference to the following examples. These examples are meant to be illustrative of pharmaceutical compositions and dosage forms provided herein, but are not in any way limiting.

### 6.1 Example 1: High Throughput In Vitro Screening

To identify a target for cancer therapy, *i.e.*, molecules implicated in regulating the inhibitory mechanisms of suppressor cells, naïve T cells are isolated from a non-tumor bearing genetically or non-genetically engineered mouse and placed into a 96 well plate. The naive T cells are engineered to contain a knocked-down or knocked-out particular gene of interest by infecting T cells using lentivirus vectors that contain a shRNA of interest as described above, or by using systems that can knockout the gene of interest such as the TALEN or CRISPR/CAS9 systems, also described above. The knock-down or knock-out of a particular candidate gene is done one well at a time.

After acquiring a stable phenotype, the shRNA treated T cells are incubated with the suppressor cells in the presence of antigen or anti-CD3 + anti-CD28, using two sets of suppressor cells: (1) suppressor cells isolated from an irradiated animal; and (2) suppressor cells isolated from a unirradiated animal. Then T-cell proliferation will be assessed in individual wells by monitoring 3[H]-thymidine incorporation using the procedures substantially similar to those described in Dolcetti et al., Current Protocols in Immunlogy, 14.17.1-14.17.25 (2010), incorporated herein by reference.

It is expected that proliferation will be suppressed in T cells treated with non-target control shRNAs whereas inactivation of target genes that negatively regulate (inhibit) the immune response will result in an enhanced response (reduced suppression). Further, the responses of T cells and/or the suppressor cells isolated from irradiated vs. unirradiated animals are compared in the same in vitro suppression assay. It is expected that significantly better T cell proliferation (i.e., reduced T cell suppression) is observed in samples that contain knock-out or knock-down of genes that are involved in inhibition of T cell responses, when combined with suppressor cells isolated from an irradiated animal. Such genes are identified as targets for cancer therapy, in particular, as targets whose inhibition can potentially induce an abscopal effect.

### 6.2 Example 2: Pooled In Vitro Screening

Another in vitro approach could essentially use the same methods as described above, except not done in an arrayed format in 96 well plates but done as pools of T-cells as a co-culture with suppressor cells. T cells are infected with lentivirus bearing the vectors that could mediate the knock-down or knock-out of candidate genes, and exactly only one cell will have only one gene that is attenuated. Each gene will have a representation of at least 500-1000 cells. A T cell suppression assay is carried out as a pool, so that any cells that can escape the suppression and proliferate in the suppressive microenvironment will be enriched. The cells bearing the vector of interest will have integrated into the genome, and therefore sequencing the vector will determine the relative representation of each vector in the pool, and any overrepresentation (or underrepresentation) of a particular vector is determined to assess the involvement of the candidate gene in inhibitory effects on T cell immunity.

### 6.3 Example 3: In Vivo Screening for Targets for Inducing Abscopal Effects

The function of candidate genes, in some instances, those candidate genes identified from in vitro assays described above, as the mediators of the abscopal effect is tested in an in vivo system where two tumors are implanted distally in the animal and only one tumor will be irradiated. The anti-tumor effect on the unirradiated tumor will be measured. If the gene is important to mediate the immunosuppressive response with irradiation, knocking it down or out in the T cells will greatly enhance the anti-tumor effect of the untreated tumor.

Naïve T cells are isolated from wild type or TCR engineered mice, and transduced with the lentiviral vector of interest using a pooled lentiviral approach, similar to the *in vitro* pooled screen approach as described above. After stable transduction of the vector of interest into the T cells, at a representation of 500-1000 for each gene, these cells are be injected back into the dual-tumor bearing mice.

For one set of animals, only one of the two tumors is irradiated at a single dose of radiation from 1 to 20 Gy. For a second set of animals, the tumors are not irradiated. After a period of 7-20 days following irradiation, transduced T cells (which can be identified by expression of a specific congenic marker on the cell surface that is not expressed by endogenous T cells from the tumor bearing animal, or that is co-expressed by the vector that is introduced into the cell) are isolated from draining lymph node, non-draining lymph node, spleen, and the unirradiated tumor.

Genomic DNA from the T cells is extracted, and the specific vectors are amplified and sequenced using next generation sequencing. T cells containing candidate genes that are involved in induction of the abscopal effect are expected to show enhanced proliferation. It is expected that T cell activation and expansion only occurs when both the gene of interest is knocked down or out and in the presence of ablative irradiation of the primary tumor. Thus, this activation should be evident in the T cells infiltrating the untreated tumor and also in the shrinkage of the untreated tumor when the opposite tumor is irradiated and the gene of interested is knocked down. In conatrast, T cell activation is expected to be low or negligible in the unirradiated mouse.

### 6.4 Example 4: In vivo shRNA Screening

An *in vivo* screen in a two-tumor mouse model was performed targeting G protein-coupled receptors (GPCRs), tumor necrosis factor receptor superfamily (TNFRSF), and immunoglobulin super family library (IgSF). A total of 134 candidate genes were screened, represented by 650 shRNA close, with each candidate gene of these families was represented by 4 to 5 shRNAs. For a number of genes, shRNAs were over-represented in test tissues comparing to the starting T-cell population, indicating enhanced proliferation and accumulation of the T-cell clone incorporating the shRNA, independent of the TCR recognition of a tumor antigen, and when one tumor of the test subject received targeted radiation. Thus, these genes were identified as targets for cancer therapy, either alone or in combination with radiation. The validity of these identified targets can be further corroborated with additional testing. On the other hand, genes with a selective loss of shRNAs in the test tissue indicate that these genes likely play important roles in T-cell activation and inhibition.

*Construction of Pooled shRNA Library.* Three shRNAs pools targeting G protein-coupled receptors (GPCRs), tumor necrosis factor receptor superfamily (TNFRSF), and immunoglobulin super family library (IgSF) were created by subcloned 4 to 5 shRNA against each target gene. All of the shRNA constructs were designed and constructed by ligating annealed oligonucleotides into the pLKO.3-Thy1.1, which coexpresses Thy1.1 and shRNA by lentiviral transduction in mouse naive CD8+ T cells. Each shRNAs pool containing 82 negative-control shRNAs, including 21 green fluorescent protein (GFP), 16 red fluorescent protein (RFP), 25 luciferase and 20 *LacZ.*

*Lentiviral transduction of primary mouse naïve CD8*+ *T cells.* The primary mouse naive CD8+ T cells were isolated from OT-1 transgenic mouse (C57BL/6-Tg (TcraTcrb) 1100Mjb/J, The Jackson laboratory) by using mouse naive CD8+ T cell isolation kit (Miltenyi Biotec), and transduced with lentiviral pool at low multiplicity of infection (MOIs). Before lentiviral transduction, isolated naive CD8+ T cells were cultured with IL-7 (5 ng/ml) and IL 15 (100 ng/ml) in complete RPMI media (RPMI 1640, 10% FBS, 20 mM HEPES, 1 mM sodium pyruvate, 0.05 mM 2-mercapto-ethanol, 2 mM L-glutamine, 100 µg/ml streptomycin and 100 µg/ml penicillin) for 48 hours. Seeding CD8+ T cells (8 x 10⁶ cells per well) with lentiviral pools supplemented with protamine sulphate (5 µg/ml) into RetroNectin reagent precoated 6-well plate (5 µg/ml) and infected by spinoculation at an MOI of 25 to 50. OT-I CD8+ T cells were further cultured in complete RPMI media containing IL-7 (2.5 ng /ml), IL-15 (50 ng /ml) and IL-2 (2 ng ml) follow by lentiviral infection. At 48 hours post-infection, virus infectivity was examined by Thy1.1 expression, which was analyzed by flow cytometry using anti-Thy1.1-PE antibody.

*Treatment of tumors by adoptive transfer of CD8*+ *T cells into tumor bearing mice.* B16-Ova cells (1.5 × 10⁵ cells in 50 µl) were inoculated by epidermal injection into male C57BL/6 mice (10 weeks of age) and measured tumor size every three days following inoculation and calculated as length x width. On day 9, 30 tumors bearing mice with similar tumor size on leg and Hank were divided into 2 groups as non-irradiation (non-IR) and irradiation (IR). The irradiation group was treated with 12Gy IR on right leg tumor using a shepherd Cesium irradiator at Day 9. On day 11, the shRNA pool infected Thy1.1 positive CD8+ T cells were isolated by PE-positive selection using Thy1.1 as selection marker (EasySep™ PE positive selection kit, Stem Cells Technologies) at 72 hours post-infection. The isolated CD8+ T cells with 90% Thy1.1 positivity (5 × 10⁶ cells in 200 ul) were injected into B16-Ova tumors bearing C57BL/6 mice by retro-orbital injection. Tumor size was measured by every two days following transfer and calculated as length x width. Mice with tumors ≥ 20 mm on the longest axis were euthanized.

*Isolate Thy1.1 positivie CD8*+ *T cells from adoptive transfer tumors bearing C57BL*/*6 mice.* The lymphocytes were isolated from the spleens, draining lymph nodes, non-draining lymph nodes of non-IR and IR mice. The tumor-infiltrated lymphocytes (TILs) were purified from leg and flank tumors, B16-Ova tumors were taken from mice and washed with PBS with 2% FBS. Resuspended the tumors in 15 ml RPMI containing 2% FBS, 50 U/ml collagenase type IV (Invitrogen) and 20 U/ml DNaseI (Roche) and incubated at 37 °C for 2 h. TILs were further isolated by using Lympholyte-M (Cedarlane Laboratories). The purified lymphocytes were fluorescently labeled using anti-Vα2-FITC- anti-Thy1.1-PE, anti-β5V-PerCP, anti-CD45-APC and anti-CD8-eFlour450 antibodies. The shRNA-expressing Thy1.1 positive CD8+ T cells (CD8⁺Vα2⁺Vβ5⁺Thy1.1⁺) were sorted by fluorescence activated cell sorting (FACS) from each samples using a FACSAria (BD Biosciences).

*Deep sequencing using the Illumina MiSeq genetic Analyzer.* The genomic DNA of collected samples was isolated (DNeasy blood & tissue kit, QIAGEN) and served as the template to generate PCR amplicons from shRNA cassette for deep-sequencing assay. Representation of shRNAs in each pool was analyzed by deep sequencing using the MiSeq from Illumina. Data were normalized using the average reads of control shRNAs in each pool.

*Statistical analysis of deep sequencing data.* The reads were mapped to FASTA file of pool shRNA library using Bowtie2. To avoid dividing by zero in calculation of fold change, 1 unit was added to the raw count for each clone. Then, for each sample, the count data were normalized to the total number of reads of that sample. The normalized read count for each clone was represented as Reads-per-Million ("RPM") reads. The normalized read counts were used to calculate fold change.

As shown below in Tables 1-3, during this screen, which covered 134 genes (650 shRNA clones, a number of gene targets were identified. For example, 22 target genes were identified that the expression levels of two associated shRNA clones were more than 2 fold higher in Thy1.1 positive CD8+ T cells obtained from the leg tumor compared to those obtained from the spleen from the mice that received irradiation in their leg tumors.

**Table 1: Fold Difference in Expression of shRNAs from T cells: Non-Irradiated Leg Tumor versus Non-Irradiated Spleen**

| **Fold of Difference** | **# of Targets with 4 shRNA clones identified** | **# of Targets with 3 shRNA clones identified** | **# of Targets with 2 shRNA clones identified** |
|---|---|---|---|
| More than 4 Fold (NR-Leg/NR-Spleen) | 3 | 5 | 22 |
| About 2 to 4 Fold (NR-Leg/NR-Spleen) | 1 | 5 | 10 |
| About 2 Fold (NR-Leg/NR-Spleen) | 4 | 11 | 28 |

| | | | |
|---|---|---|---|
| NR: Non-Irradiated NR-Leg: Tumor from non-irradiated leg NR-Spleen: Spleen from non-irradiated mouse *Only leg tumor is irradiated, not flank tumor | | | |

**Table 2: Fold Difference in Expression of shRNAs from T cells: Non-Irradiated Flank Tumor versus Non-Irradiated Spleen**

| **Fold of Difference** | **# of Targets with 4 shRNA clones identified** | **# of Targets with 3 shRNA clones identified** | **# of Targets with 2 shRNA clones identified** |
|---|---|---|---|
| More than 4 Fold (NR-Flank/NR-Spleen) | 0 | 1 | 7 |
| About 2 to 4 Fold (NR-Leg/NR-Spleen) | 1 | 3 | 12 |
| About 2 Fold (NR-Leg/NR-Spleen) | 0 | 7 | 16 |

| | | | |
|---|---|---|---|
| NR: Non-Irradiated NR-Leg: Tumor from non-irradiated leg NR-Spleen: Spleen from non-irradiated mouse *Only leg tumor is irradiated, not flank tumor | | | |

**Table 3: Fold Difference in Expression of shRNAs from T cells: Irradiated Leg Tumor versus Spleen with leg tumor irradiated**

| **Fold of Difference** | **# of Targets with 4 shRNA clones identified** | **# of Targets with 3 shRNA clones identified** | **# of Targets with 2 shRNA clones identified** |
|---|---|---|---|
| More than 4 Fold (R-Leg/R-Spleen) | 0 | 0 | 6 |
| About 2 to 4 Fold (R-Leg/R-Spleen) | 0 | 0 | 5 |
| About 2 Fold (R-Leg/R-Spleen) | 0 | 3 | 22 |

| | | | |
|---|---|---|---|
| R-Leg: Tumor from irradiated on leg tumor R-Spleen: Spleen from mouse irradiated on leg tumor *Only leg tumor is irradiated, not flank tumor | | | |

## Claims

1. An *in vitro* method of identifying a target gene for cancer therapy comprising measuring an activity of an unmodified T cell and of a modified T cell; said unmodified T cell and said modified T cell each contacted with the tumor microenvironment from an irradiated tumor-bearing subject; said modified T cell having a candidate gene knocked-down or knocked-out; wherein said candidate gene is identified as a target gene for cancer therapy if said activity is increased comparing said modified T cell to said unmodified T cell; and wherein said activity is selected from the group consisting of proliferation, cell cycle activation, cell death inhibition, cytokine production, and a cytotoxic activity.

2. The method of claim 1, further comprising measuring said activity of said unmodified T cell and of said modified T cell each contacted with the tumor microenvironment from a non-irradiated tumor-bearing subject; wherein said activity in said unmodified T cell and said modified T cell are substantially the same.

3. An *in vivo* method of identifying a target gene for cancer therapy comprising:
(i) injecting unmodified T cell and modified T cell to irradiated tumor-bearing non-human test subjects; said modified T cell having a candidate gene knocked-down or knocked-out; and
(ii) measuring an activity of said unmodified T cell and of said modified T cell in said non-human test subjects, wherein said activity is proliferation activity or anti-tumor activity;
wherein said candidate gene is identified as a target gene for cancer therapy if said activity is increased comparing said modified T cell to said unmodified T cell.

4. The method of claim 3, wherein said modified T cell is a reference T cell.

5. The method of claim 3 or 4, further comprising injecting said unmodified T cell and said modified T cell to non-irradiated test subjects, wherein said activity is substantially the same comparing said modified T cell to said unmodified T cell in said non-irradiated test subjects.

6. The method of any one of claims 1 to 5, wherein said subject has received tumor-targeted radiation at a dose from 1 Gy to 20 Gy.

7. The method of any one of claims 3 to 6, wherein said activity is:
(i) proliferation activity measured by accumulation of injected T cells;
(ii) anti-tumor activity measured by death of tumor cells or the reduction of tumor size; or
(iii) anti-tumor activity measured by increased survival of tested subjects.

8. The method of any one of claims 3 to 7, wherein said test subject is a mouse, preferably wherein said mouse is a two-tumor mouse, wherein only one tumor has received targeted radiation.

9. The method of claim 8, wherein said activity is anti-tumor activity measured by size reduction of the non-irradiated tumor.

10. An *in vitro* pool screen method of identifying a target gene for cancer therapy comprising:
(i) preparing a starting population of T cells with different genetic modifications, wherein each T cell has at most one candidate gene knocked-down or knocked-out; and
(ii) contacting said starting population of T cells with the tumor microenvironment from an irradiated tumor-bearing subject for a period of time to produce a selected population of T cells;
wherein the candidate gene that is knocked-down or knocked-out in the selected population of T cells is identified to be the target gene for cancer therapy.

11. An *in vivo* pool screen method of identifying a target gene for cancer therapy comprising:
(i) preparing a starting population of T cells with different genetic modifications, wherein each T cell has at most one candidate gene knocked-down or knocked-out; and
(ii) injecting said starting population of T cells to an irradiated tumor-bearing non-human test subject, in which said starting population of T cells produces a selected population of T cells;
wherein the candidate gene that is knocked-down or knocked-out in the selected population of T cells is identified to be the target gene for cancer therapy.

12. The method of any one of claims 1 to 11, wherein said candidate gene is knocked-down or knocked-out in said modified T cell, or said starting population of T cells by:
(i) using a transcription activator-like effector nuclease (TALEN);
(ii) using CAS9 (CRISPR); or
(iii) infecting T cells with a shRNA library, optionally wherein the shRNA library is a lentivirus vector library.

13. The method of any one of claims 1 to 11, wherein said T cells or said starting population of T cells are:
(i) naive T cells;
(ii) CD8+ T cells; or
(iii) isolated from a mouse, optionally wherein said mouse is wild type, or genetically engineered to express a T-cell receptor specific for an antigen not naturally present in mice, optionally wherein said antigen is ovalbumin.

14. The method of any one of claims 1 to 11, wherein said T cells or said starting population of T cells are labeled, optionally wherein said label is carboxyfluorescein succinimidyl ester (CFSE).

15. The method of any one of claims 10 to 13, further comprising isolating said selected population of T cells from a sample of said test subject, optionally wherein:
(i) said sample is a tumor sample, optionally wherein said tumor sample has received targeted radiation or the test subject is a two-tumor model, and said tumor sample has not received targeted radiation; or
(ii) said sample is a non-tumor sample, optionally wherein said non-tumor tissue sample is a spleen sample or a lymph node sample.

16. The method of any one of claims 10 to 13, wherein the candidate gene that is knocked-down or knocked-out in the selected population of T cells is identified by NGS of genomic DNAs isolated from the selected population of T cells.

17. The method of claim 12(iii), wherein each T cell of the starting population has at most a shRNA for a candidate gene, and a candidate gene is identified to be a target gene for cancer therapy if the shRNA for the candidate gene is enriched in the selected population of T cells as compared to the starting population of T cells, optionally wherein:
(i) a candidate gene is identified to be a target gene for cancer therapy if the shRNA for the candidate gene is enriched in the selected population of T cells obtained from a tumor sample as compared to a non-tumor tissue sample;
(ii) a candidate gene is identified as a target gene if the shRNA for said candidate gene is enriched more than 2 fold in the tumor sample compared to the non-tumor tissue sample; or
(iii) a candidate gene is identified as a target gene if the shRNA for said candidate gene is enriched more than 3 fold, more than 4 fold, more than 5 fold, more than 10 fold, more than 15 fold, or more than 20 fold in the tumor sample compared to the non-tumor tissue sample.

18. The method of claim 17, wherein the method comprises quantifying shRNA in the selected population of T cells by NGS.

## Patentansprüche

1. *In vitro*-Verfahren zum Identifizieren eines Zielgens für die Krebstherapie, umfassend das Messen einer Aktivität einer nicht modifizierten T-Zelle und einer modifizierten T-Zelle; wobei die nicht modifizierte T-Zelle und die modifizierte T-Zelle jeweils mit der Tumormikroumgebung von einem bestrahlten tumortragenden Subjekt in Kontakt gebracht werden; wobei die modifizierte T-Zelle ein Kandidatengen aufweist, bei dem ein Knock-down oder Knock-out erfolgt ist; wobei das Kandidatengen als ein Zielgen für die Krebstherapie identifiziert wird, wenn die Aktivität bei Vergleich der modifizierten T-Zelle mit der nicht modifizierten T-Zelle erhöht ist; und wobei die Aktivität ausgewählt ist aus der Gruppe, die aus Proliferation, Zellzyklusaktivierung, Zelltodinhibition, Zytokinproduktion und einer zytotoxischen Aktivität besteht.

2. Verfahren nach Anspruch 1, ferner umfassend das Messen der Aktivität der nicht modifizierten T-Zelle und der modifizierten T-Zelle, die jeweils mit der Tumormikroumgebung von einem nicht bestrahlten tumortragenden Subjekt in Kontakt gebracht werden; wobei die Aktivität in der nicht modifizierten T-Zelle und der modifizierten T-Zelle im Wesentlichen die gleiche ist.

3. *In vivo*-Verfahren zum Identifizieren eines Zielgens für die Krebstherapie, umfassend:
(i) Injizieren einer nicht modifizierten T-Zelle und einer modifizierten T-Zelle in bestrahlte tumortragende nicht menschliche Versuchssubjekte; wobei die modifizierte T-Zelle ein Kandidatengen aufweist, bei dem ein Knockdown oder Knockout erfolgt ist; und
(ii) Messen einer Aktivität der nicht modifizierten T-Zelle und der modifizierten T-Zelle in den nicht menschlichen Versuchssubjekten, wobei die Aktivität Proliferationsaktivität oder Antitumoraktivität ist,
wobei das Kandidatengen als ein Zielgen für die Krebstherapie identifiziert wird, wenn die Aktivität bei Vergleich der modifizierten T-Zelle mit der nicht modifizierten T-Zelle erhöht ist.

4. Verfahren nach Anspruch 3, wobei die modifizierte T-Zelle eine Referenz-T-Zelle ist.

5. Verfahren nach Anspruch 3 oder 4, ferner umfassend das Injizieren der nicht modifizierten T-Zelle und der modifizierten T-Zelle in nicht bestrahlte Versuchssubjekte, wobei die Aktivität bei Vergleich der modifizierten T-Zelle mit der nicht modifizierten T-Zelle in den nicht bestrahlten Versuchssubjekten im Wesentlichen die gleiche ist.

6. Verfahren nach einem der Ansprüche 1 bis 5, wobei das Subjekt eine auf den Tumor gezielte Strahlung in einer Dosis von 1 Gy bis 20 Gy erhalten hat.

7. Verfahren nach einem der Ansprüche 3 bis 6, wobei die Aktivität folgende ist:
(i) Proliferationsaktivität, gemessen durch Ansammlung injizierter T-Zellen;
(ii) Antitumoraktivität, gemessen durch Tumorzelltod oder Abnahme der Tumorgröße; oder
(iii) Antitumoraktivität, gemessen durch erhöhtes Überleben der Versuchssubjekte.

8. Verfahren nach einem der Ansprüche 3 bis 7, wobei das Versuchssubjekt eine Maus ist, wobei die Maus vorzugsweise eine Zweitumormaus ist, wobei nur ein Tumor gezielte Strahlung erhalten hat.

9. Verfahren nach Anspruch 8, wobei die Aktivität Antitumoraktivität, gemessen anhand der Größenabnahme des nicht bestrahlten Tumors, ist.

10. *In vitro*-Pool-Analyse-Verfahren zum Identifizieren eines Zielgens für die Krebstherapie, umfassend:
(i) Herstellen einer Ausgangspopulation von T-Zellen mit unterschiedlichen genetischen Modifikationen, wobei jede T-Zelle höchstens ein Kandidatengen aufweist, bei dem ein Knockdown oder Knockout erfolgt ist; und
(ii) In-Kontakt-Bringen der Ausgangspopulation von T-Zellen mit der Tumormikroumgebung von einem bestrahlten tumortragenden Subjekt über einen Zeitraum, um eine ausgewählte Population von T-Zellen zu produzieren;
wobei das Kandidatengen in der ausgewählten Population von T-Zellen, bei dem ein Knockdown oder Knockout erfolgt ist, als das Zielgen für die Krebstherapie identifiziert wird.

11. *In v*i*vo-*Pool-Analyse-Verfahren zum Identifizieren eines Zielgens für die Krebstherapie, umfassend:
(i) Herstellen einer Ausgangspopulation von T-Zellen mit unterschiedlichen genetischen Modifikationen, wobei jede T-Zelle höchstens ein Kandidatengen aufweist, bei dem ein Knockdown oder Knockout erfolgt ist; und
(ii) Injizieren der Ausgangspopulation von T-Zellen in ein bestrahltes tumortragendes nicht menschliches Versuchssubjekt, wobei die Ausgangspopulation von T-Zellen eine ausgewählte Population von T-Zellen produziert;
wobei das Kandidatengen in der ausgewählten Population von T-Zellen, bei dem ein Knockdown oder Knockout erfolgt ist, als das Zielgen für die Krebstherapie identifiziert wird.

12. Verfahren nach einem der Ansprüche 1 bis 11, wobei in der modifizierten T-Zelle oder der Ausgangspopulation von T-Zellen durch Folgendes ein Knockdown oder Knockout des Kandidatengens erfolgt:
(i) Verwendung einer transkriptionsaktivatorartigen Effektornuklease (TALEN);
(ii) Verwendung von CAS9 (CRISPR); oder
(iii) Infizieren von T-Zellen mit einer shRNA-Bibliothek, wobei die shRNA-Bibliothek wahlweise eine Lentivirusvektorbibliothek ist.

13. Verfahren nach einem der Ansprüche 1 bis 11, wobei die T-Zellen oder die Ausgangspopulation von T-Zellen Folgendes sind:
(i) naive T-Zellen;
(ii) CD8+-T-Zellen; oder
(iii) aus einer Maus isoliert, wobei die Maus wahlweise ein Wildtyp ist, oder gentechnisch hergestellt, um einen T-Zell-Rezeptor zu exprimieren, der für ein Antigen spezifisch ist, das in Mäusen natürlicherweise nicht vorhanden ist, wobei das Antigen wahlweise Ovalbumin ist.

14. Verfahren nach einem der Ansprüche 1 bis 11, wobei die T-Zellen oder die Ausgangspopulation von T-Zellen markiert sind, wobei die Markierung wahlweise Carboxyfluoresceinsuccinimidylester (CFSE) ist.

15. Verfahren nach einem der Ansprüche 10 bis 13, ferner umfassend das Isolieren der ausgewählten Population von T-Zellen aus einer Probe des Versuchssubjekts, wobei wahlweise
(i) die Probe eine Tumorprobe ist, wobei die Tumorprobe wahlweise gezielte Strahlung erhalten hat oder das Versuchssubjekt eine Zweitumormaus ist, und die Tumorprobe keine gezielte Strahlung erhalten hat; oder
(ii) die Probe eine Nichttumorprobe ist, wobei die Nichttumorgewebeprobe wahlweise eine Milzprobe oder eine Lymphknotenprobe ist.

16. Verfahren nach einem der Ansprüche 10 bis 13, wobei das Kandidatengen in der ausgewählten Population von T-Zellen, bei dem ein Knockdown oder Knockout erfolgt ist, mittels NGS der aus der ausgewählten Population von T-Zellen isolierter genomischen DNA identifiziert wird.

17. Verfahren nach Anspruch 12(iii), wobei jede T-Zelle der Ausgangspopulation höchstens eine shRNA für ein Kandidatengen aufweist und ein Kandidatengen als ein Zielgen für die Krebstherapie identifiziert wird, sofern die shRNA für das Kandidatengen in der ausgewählten Population von T-Zellen im Vergleich zu der Ausgangspopulation von T-Zellen angereichert ist, wobei wahlweise
(i) ein Kandidatengen als ein Zielgen für die Krebstherapie identifiziert wird, wenn die shRNA für das Kandidatengen in der ausgewählten Population von T-Zellen, gewonnen aus von einer Tumorprobe, im Vergleich zu einer Nichttumorgewebeprobe angereichert ist;
(ii) ein Kandidatengen als ein Zielgen identifiziert wird, wenn die shRNA für das Kandidatengen in der Tumorprobe im Vergleich zu der Nichttumorgewebeprobe um mehr als das 2-Fache angereichert ist; oder
(iii) ein Kandidatengen als ein Zielgen identifiziert wird, wenn die shRNA für das Kandidatengen in der Tumorprobe im Vergleich zu der Nichttumorgewebeprobe um mehr als das 3-Fache, mehr als das 4-Fache, mehr als das 5-Fache, mehr als das 10-Fache, mehr als das 15-Fache oder mehr als das 20-Fache angereichert ist.

18. Verfahren nach Anspruch 17, wobei das Verfahren das Quantifizieren von shRNA in der ausgewählten Population von T-Zellen mittels NGS umfasst.

## Revendications

1. Procédé *in vitro* d'identification d'un gène cible pour une thérapie anticancéreuse comprenant la mesure d'une activité d'un lymphocyte T non modifié et d'un lymphocyte T modifié ; ledit lymphocyte T non modifié et ledit lymphocyte T modifié étant chacun mis en contact avec le micro-environnement tumoral d'un sujet portant une tumeur irradiée ; ledit lymphocyte T modifié ayant un gène candidat knocked-down ou knocked-out ; dans lequel ledit gène candidat est identifié comme un gène cible de thérapie anticancéreuse si ladite activité est accrue en comparaison dudit lymphocyte T modifié par rapport audit lymphocyte T non modifié ; et dans lequel ladite activité est sélectionnée dans le groupe consistant en prolifération, activation du cycle cellulaire, inhibition de la mort cellulaire, production de cytokine et une activité cytotoxique.

2. Procédé selon la revendication 1, comprenant en outre la mesure de ladite activité dudit lymphocyte T non modifié et dudit lymphocyte T modifié, chacun étant mis en contact avec le micro-environnement tumoral d'un sujet portant une tumeur non irradiée ; dans lequel ladite activité dans ledit lymphocyte T non modifié et ledit lymphocyte T modifié est sensiblement la même.

3. Procédé *in vivo* d'identification d'un gène cible pour une thérapie anticancéreuse comprenant :
(i) l'injection d'un lymphocyte T non modifié et d'un lymphocyte T modifié dans des sujets de test non humains portant une tumeur irradiée ; ledit lymphocyte T modifié ayant un gène candidat knocked-down ou knocked-out ; et
(ii) la mesure d'une activité dudit lymphocyte T non modifié et dudit lymphocyte T modifié dans lesdits sujets de test non humains, dans lequel ladite activité est une activité de prolifération ou une activité antitumorale ;
dans lequel ledit gène candidat est identifié comme gène cible pour une thérapie anticancéreuse si ladite activité est accrue par comparaison dudit lymphocyte T modifié audit lymphocyte T non modifié.

4. Procédé selon la revendication 3, dans lequel ledit lymphocyte T modifié est un lymphocyte T de référence.

5. Procédé selon la revendication 3 ou 4, comprenant en outre l'injection dudit lymphocyte T non modifié et dudit lymphocyte T modifié à des sujets de test non irradiés, dans lequel ladite activité est sensiblement la même par comparaison dudit lymphocyte T modifié audit lymphocyte T non modifié dans lesdits sujets de test non irradiés.

6. Procédé selon l'une quelconque des revendications 1 à 5, dans lequel ledit sujet a reçu un rayonnement ciblé sur la tumeur à une dose de 1 Gy à 20 Gy.

7. Procédé selon l'une quelconque des revendications 3 à 6, dans lequel ladite activité est :
(i) une activité de prolifération mesurée par l'accumulation de lymphocytes T injectés;
(ii) une activité antitumorale mesurée par la mort de cellules tumorales ou la réduction de la taille de la tumeur; ou
(iii) une activité antitumorale mesurée par l'augmentation de la survie de sujets testés.

8. Procédé selon l'une quelconque des revendications 3 à 7, dans lequel ledit sujet de test est une souris, de préférence dans lequel ladite souris est une souris ayant deux tumeurs, dans lequel seule une tumeur a reçu un rayonnement ciblé.

9. Procédé selon la revendication 8, dans lequel ladite activité est une activité antitumorale mesurée par une réduction de taille de la tumeur non irradiée.

10. Procédé de criblage *in vitro* d'un groupe pour identifier un gène cible pour une thérapie anticancéreuse comprenant:
(i) la préparation d'une population de départ de lymphocytes T ayant différentes modifications génétiques, dans lequel chaque lymphocyte T a au plus un gène candidat knocked-down ou knocked-out; et
(ii) la mise en contact de ladite population de départ de lymphocytes T avec le micro-environnement tumoral d'un sujet portant une tumeur irradiée pendant un certain temps pour produire une population de lymphocytes T sélectionnée;
dans lequel le gène candidat qui est knocked-down ou knocked-out dans la population sélectionnée de lymphocytes T est identifié comme étant le gène cible pour une thérapie anticancéreuse.

11. Procédé de criblage *in vivo* d'un groupe pour identifier un gène cible pour une thérapie anticancéreuse comprenant:
(i) la préparation d'une population de départ de lymphocytes T ayant des modifications génétiques différentes, dans lequel chaque lymphocyte T a au plus un gène candidat knocked-down ou knocked-out; et
(ii) l'injection de ladite population de départ de lymphocytes T à un sujet de test non humain portant une tumeur irradiée, dans lequel ladite population de départ de lymphocytes T produit une population sélectionnée de lymphocytes T;
dans lequel le gène candidat qui est knocked-down ou knocked-out dans la population sélectionnée de lymphocytes T est identifié comme étant le gène cible d'une thérapie anticancéreuse.

12. Procédé selon l'une quelconque des revendications 1 à 11, dans lequel ledit gène candidat est un gène knocked-down ou knocked-out dans ledit lymphocyte T modifié, ou ladite population de départ de lymphocytes T par:
(i) l'utilisation d'une nucléase effectrice de type activateur de transcription (TALEN);
(ii) l'utilisation de CAS9 (CRISPR); ou
(iii) l'infection de lymphocytes T avec une bibliothèque d'ARNsh, éventuellement dans lequel la bibliothèque d'ARNsh est une bibliothèque de vecteurs lentivirus.

13. Procédé selon l'une quelconque des revendications 1 à 11, dans lequel lesdits lymphocytes T ou ladite population de départ des lymphocytes T sont:
(i) des lymphocytes T naïfs;
(ii) des lymphocytes T CD8+; ou
(iii) isolés à partir d'une souris, éventuellement dans lequel ladite souris est de type sauvage, ou génétiquement modifiée pour exprimer un récepteur de lymphocyte T spécifique d'un antigène qui n'est pas présent à l'état naturel chez les souris, éventuellement dans lequel ledit antigène est l'ovalbumine.

14. Procédé selon l'une quelconque des revendications 1 à 11, dans lequel lesdits lymphocytes T ou ladite population de départ des lymphocytes T sont marqués, éventuellement dans lequel ledit marqueur est un ester de carboxyfluorescéine succinimidyle (CFSE).

15. Procédé selon l'une quelconque des revendications 10 à 13, comprenant en outre l'isolement de ladite population sélectionnée de lymphocytes T à partir d'un échantillon dudit sujet de test, éventuellement dans lequel:
(i) ledit échantillon est un échantillon tumoral, éventuellement dans lequel ledit échantillon tumoral a reçu un rayonnement ciblé ou le sujet de test est un modèle ayant deux tumeurs, et ledit échantillon de tumeur n'a pas reçu de rayonnement ciblé; ou
(ii) ledit échantillon est un échantillon non tumoral, éventuellement dans lequel ledit échantillon de tissu non tumoral est un échantillon de rate ou un échantillon de ganglion lymphatique.

16. Procédé selon l'une quelconque des revendications 10 à 13, dans lequel le gène candidat qui est knocked-down ou knocked-out dans la population sélectionnée de lymphocytes T est identifié par NGS d'ADN génomiques isolés dans la population sélectionnée de lymphocytes T.

17. Procédé selon la revendication 12 (iii), dans lequel chaque lymphocyte T de la population de départ a au plus un ARNsh pour un gène candidat, et un gène candidat est identifié comme étant un gène cible pour une thérapie anticancéreuse si l'ARNsh pour le gène candidat est enrichi dans la population sélectionnée de lymphocytes T par comparaison à la population de départ de lymphocytes T, éventuellement dans lequel :
(i) un gène candidat est identifié comme étant un gène cible pour une thérapie anticancéreuse si l'ARNsh du gène candidat est enrichi dans la population sélectionnée de lymphocytes T obtenus à partir d'un échantillon tumoral par comparaison à un échantillon de tissu non tumoral ;
(ii) un gène candidat est identifié comme étant un gène cible si l'ARNsh dudit gène candidat est enrichi plus de 2 fois dans l'échantillon tumoral par comparaison à l'échantillon de tissu non tumoral ; ou
(iii) un gène candidat est identifié comme étant un gène cible si l'ARNsh dudit gène candidat est enrichi plus de 3 fois, plus de 4 fois, plus de 5 fois, plus de 10 fois, plus de 15 fois, ou plus de 20 fois dans l'échantillon tumoral par comparaison à l'échantillon de tissu non tumoral.

18. Procédé selon la revendication 17, dans lequel le procédé comprend la quantification de l'ARNsh dans la population sélectionnée de lymphocytes T par NGS.
